# EUROPEAN PATENT APPLICATION

(11) **EP 4 509 596 A1**
(43) Date of publication of application: **19.02.2025**
(21) Application number: 23788063.8
(22) Date of filing: 03.03.2023
(51) Int. Cl.: C12N 5/0775, C12N 5/0783, C12N 1/04

(54) **MAMMALIAN CELL CRYOPRESERVATION LIQUID**

(30) Priority: 12.04.2022 JP 2022065637
(71) Applicant: Otsuka Pharmaceutical Factory, Inc., Naruto-shi, Tokushima 772-8601 (JP)
(72) Inventor: NISHIMURA, Masuhiro, Naruto-shi, Tokushima 772-8601 (JP); KOMORI, Natsuki, Naruto-shi, Tokushima 772-8601 (JP)
(74) Representative: Script Intellectual Property LLP
(86) International application number: PCT/JP2023/008060
(87) International publication number: WO 2023/199641

(57) **Abstract**

It is to provide a liquid for cryopreserving a cell that can effectively suppress cell death caused by freezing and thawing of a mammalian cell and that can effectively increase the proliferation ability of the mammalian cell after freezing and thawing, and a method for cryopreserving a mammalian cell using the liquid for cryopreserving a cell. A liquid comprising 2.5 to 8.75 (v/v)% propylene glycol is used as a liquid for cryopreserving a mammalian cell.

## Description

### Technical Field

The present invention relates to a liquid for cryopreserving a mammalian cell, comprising 2.5 to 8.75 (v/v)% propylene glycol (hereinafter sometimes referred to as "PG") (hereinafter sometimes referred to as the "present cryopreservation liquid"), a method for cryopreserving a mammalian cell, comprising a step (a) of cryopreserving the present cryopreservation liquid comprising a mammalian cell (hereinafter sometimes referred to as the "present cryopreservation method"), and the like.

### Background Art

Cryopreservation of a cell has been widely used as an essential technology in cell biology research. In recent years, a cell cryopreservation technology has been applied not only to the preservation of various established cell lines in cell banks around the world, but also to the preservation of species in the livestock industry, the cryopreservation of a sperm, an egg, or a fertilized egg for increasing livestock production, the cryopreservation of a germ cell in reproductive medicine, and the like.

A pluripotent stem cell, such as an Embryonic Stem cell (ES cell) or an induced Pluripotent Stem cell (iPS cell), is a cell having an unlimited proliferative capacity and the capacity for pluripotent differentiation into diverse tissue cells. A human pluripotent stem cell is expected to be applied to regenerative medicine by utilizing properties thereof, and in order to realize this, it is essential to establish a high-quality cell freezing technology, that is, a cell freezing technology that guarantees a high cell viability and an undifferentiated state after thawing.

Methods for cryopreserving a cell are generally divided roughly into a slow freezing method and a quick freezing method (vitrification method). The slow freezing method is a method involving suspending a cell in a cryopreservation liquid (Patent Documents 1 to 5) comprising glycerin, dimethyl sulfoxide (hereinafter sometimes referred to as "DMSO"), a keratin hydrolyzate, hydrolyzed gelatin, serum, serum albumin, or the like as a cryoprotectant, and gradually freezing the cell by lowering the temperature by about 1°C per minute. Slow cooling replaces an intracellular water molecule with the cryoprotectant and dehydrates the cell to suppress the growth of an ice crystal inside and around the cell, preventing damage to a cell membrane and an intracellular structure, and the denaturation and the cleavage of a protein (Non-patent Document 1). Recently, it has been found that when a general cell is cryopreserved, except when an embryo is cryopreserved, it is possible to slowly freeze the general cell by using a Styrofoam box or a commercially available cell freezing box without having to strictly control the temperature regulation by using a programmed freezer. Such a method is simple and thus is also called a simple slow freezing method, and is widely used in a laboratory, a cell bank, and the like.

On the other hand, the vitrification method is a method involving freezing a cell in a glassy state by quick cooling in order to suppress the formation of an ice crystal inside and outside the cell due to freezing. After the vitrification method was reported in 1937, it took a long period of technological development to put it into practical use, but in 1985, a method using a vitrification preservation liquid comprising a cryoprotectant consisting of high concentrations of DMSO, acetamide, PG, and polyethylene glycol (hereinafter referred to as "PEG") was developed. The development of this method has made it possible to cryopreserve an early mouse embryo, and cryopreserve a cow embryo and a pig embryo, which were difficult to do by using a slow freezing method. At present, the vitrification method is used in many institutions including an embryo bank.

Recently, development of a cryopreservation liquid for a human ES cell or an iPS cell, and an improvement in a cryopreservation method have been actively carried out. For example, it has been reported that the cell viability after thawing increases when a human ES cell is suspended in a cryopreservation liquid comprising 5% DMSO, 10% fetal bovine serum (FBS), and 10% ethylene glycol (EG) and cryopreserved by using a simple slow freezing method (Non-patent Document 2). In addition, it has also been reported that the cell viability after thawing increases when a human ES cell or an iPS cell is suspended in a cryopreservation liquid (STEM-CELLBANKER [manufactured by Nippon Zenyaku Kogyo Co., Ltd.]) and cryopreserved by using a simple slow freezing method (Non-Patent Document 3).

In addition, a solution for cryopreserving a cell that comprises a sugar such as trehalose and PG and is free of DMSO, a thickener, and a natural animal-derived component has been proposed (Patent Document 6). In addition, a liquid for cryopreserving an animal cell that comprises at least one selected from the group consisting of taurine, glycine, and a derivative thereof, a cryoprotectant other than DMSO, a water-soluble polysaccharide, and an oligosaccharide has also been proposed (Patent Document 7).

### Prior Art Documents

### Patent Documents

Patent Document 1: International Publication No. WO 2003/064634
Patent Document 2: Japanese unexamined Patent Application Publication No. 6-46840
Patent Document 3: Japanese unexamined Patent Application Publication No. 7-255469
Patent Document 4: Japanese unexamined Patent Application Publication No. 8-325101
Patent Document 5: Japanese unexamined Patent Application Publication No. 2002-233356
Patent Document 6: International Publication No. WO 2019/172112
Patent Document 7: Japanese unexamined Patent Application Publication No. 2021-00027

### Non-patent Documents

Non-patent Document 1: J. R. Dobrinsky, Theriogenology, 45, 17-26 (1996)
Non-patent Document 2: Y. S. Ha et al., Human Reprod., 20, 1779-1785 (2005)
Non-patent Document 3: F. Holm et al., Human Reprod., 25, 1271-1279 (2010)

### Summary of the Invention

### Object to be Solved by the Invention

An object of the present invention is to provide a liquid for cryopreserving a cell that can effectively suppress cell death caused by freezing and thawing of a mammalian cell and that can effectively increase the proliferation ability of the mammalian cell after freezing and thawing, and a method for cryopreserving a mammalian cell using the liquid for cryopreserving a cell.

### Means to Solve the Object

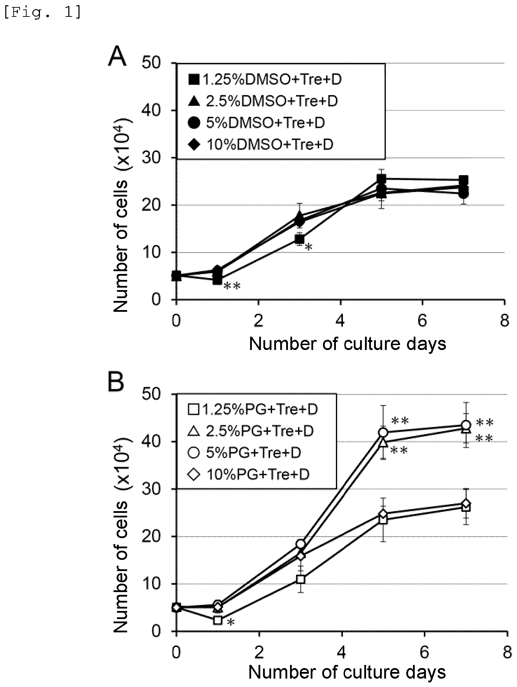

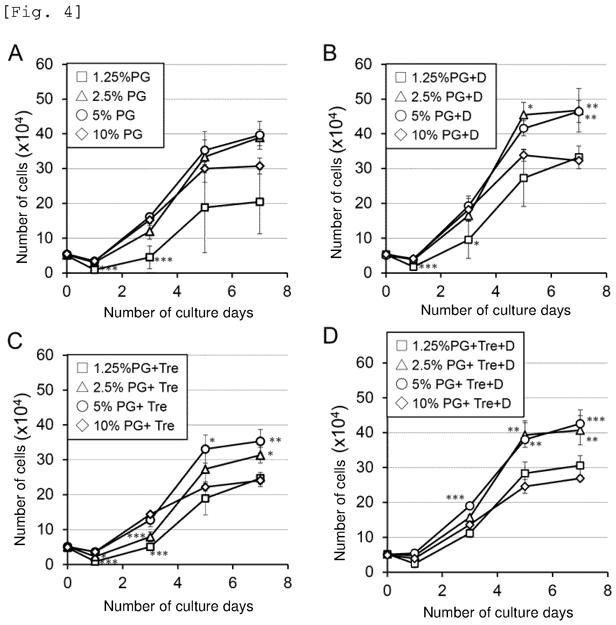

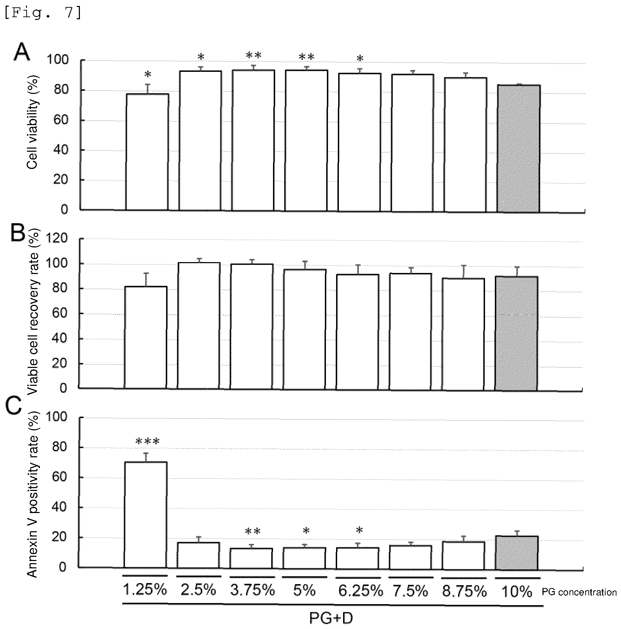

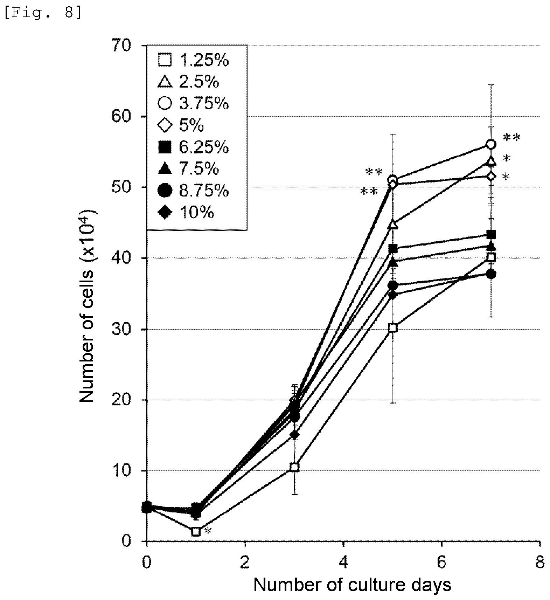

The present inventors have carried out intensive studies in order to solve the object and during the course thereof found that when a mammalian cell is cryopreserved in a liquid for cryopreserving a cell comprising 2.5 to 5% PG, the proliferation ability of the mammalian cell after freezing and thawing is higher than when a mammalian cell is cryopreserved in a liquid for cryopreserving a cell comprising 2.5 to 5% DMSO (for example, Figures 1 to 3 in an Example described later). In addition, the present inventors have confirmed that when a mammalian cell is cryopreserved in a liquid for cryopreserving a cell comprising 2.5 to 5% PG, the proliferation ability of the mammalian cell after freezing and thawing can be more effectively increased than when a mammalian cell is cryopreserved in a liquid for cryopreserving a cell comprising PG having a concentration of other than 2.5 to 5% (for example, Figure 4 and Figure 8 in an Example described later). Furthermore, the present inventors have confirmed that when a mammalian cell is cryopreserved in a liquid for cryopreserving a cell comprising 2.5 to 8.75% PG, cell death caused by freezing and thawing of the mammalian cell can be more effectively suppressed than when a mammalian cell is cryopreserved in a liquid for cryopreserving a cell comprising PG having a concentration of other than 2.5 to 8.75% (for example, Figure 7 in an Example described later). The present invention has been completed based on these findings.

Thus, the present invention is as follows.
[1] A liquid for cryopreserving a mammalian cell, comprising 2.5 to 8.75 (v/v)% propylene glycol.
[2] The liquid according to [1] above, wherein a concentration of propylene glycol is 2.5 to 5.0 (v/v)%, and wherein the liquid is used to increase a proliferation ability of a mammalian cell after freezing and thawing.
[3] The liquid according to [1] or [2] above, wherein the liquid further comprises a polymer compound selected from dextran or a derivative thereof or a salt of the dextran or the derivative; and hydroxyethyl starch or a derivative thereof or a salt of the hydroxyethyl starch or the derivative.
[4] The liquid according to any one of [1] to [3] above, wherein the liquid further comprises trehalose or a derivative thereof or a salt of the trehalose or the derivative.
[5] The liquid according to any one of [1] to [4] above, wherein the liquid is an isotonic solution comprising 2.5 to 8.75 (v/v)% propylene glycol.
[6] The liquid according to [5] above, wherein the isotonic solution is selected from lactated Ringer's solution, physiological saline, Ringer's solution, and acetated Ringer's solution.
[7] The liquid according to any one of [1] to [6] above, wherein the mammalian cell is a mesenchymal stem cell, a T cell, or a hematopoietic stem cell.
[8] The liquid according to any one of [1] to [6] above, wherein the liquid comprises a mammalian cell.
[9] The liquid according to [8] above, wherein the mammalian cell is a mesenchymal stem cell, a T cell, or a hematopoietic stem cell.
[10] The liquid according to [9] above, wherein the liquid comprises umbilical cord blood.
[11] A method for cryopreserving a mammalian cell, comprising step (a) of cryopreserving the liquid according to any one of [8] to [10] above.
[12] The method according to [11] above, wherein the method further comprises step (b) of freezing and thawing the mammalian cell and culturing the same for 5 days or more after step (a).

### Effect of the Invention

According to the present invention, when a mammalian cell is cryopreserved in a liquid for cryopreserving a cell comprising 2.5 to 8.75% PG, cell death caused by freezing and thawing of the mammalian cell can be more effectively suppressed than when a mammalian cell is cryopreserved in a liquid for cryopreserving a cell comprising PG having a concentration of other than 2.5 to 8.75%, and when a mammalian cell is cryopreserved in a liquid for cryopreserving a cell comprising 2.5 to 5% PG, the proliferation ability of the mammalian cell after freezing and thawing can be more effectively increased than when a mammalian cell is cryopreserved in a liquid for cryopreserving a cell comprising PG having a concentration of other than 2.5 to 5% or when a mammalian cell is cryopreserved in a liquid for cryopreserving a cell comprising 2.5 to 5% DMSO, thus making it possible to provide a high-quality mammalian cell-containing liquid for transplantation in regenerative medicine.

### Brief Description of Drawings

[Figure 1] Figure 1A is a diagram showing results (mean value ± SD, n = 3) of analyzing the cell proliferation ability of hAD-MSCs after thawing, the hAD-MSCs being cryopreserved for 31 days in 4 liquids for cryopreserving a cell comprising 1.25 to 10% DMSO (LR containing 1.25% DMSO, 2.7% trehalose, and 4.5% dextran ["1.25% DMSO + Tre + D" in the figure]; LR containing 2.5% DMSO, 2.7% trehalose, and 4.5% dextran ["2.5% DMSO + Tre + D" in the figure]; LR containing 5% DMSO, 2.7% trehalose, and 4.5% dextran ["5% DMSO + Tre + D" in the figure]; and LR containing 10% DMSO, 2.7% trehalose, and 4.5% dextran ["10% DMSO + Tre + D" in the figure]). "*" and "**" in the figure show that there is a statistically significant difference (p < 0.05 and p < 0.01, respectively) from "10% DMSO." Figure 1B is a diagram showing results (mean value ± SD, n = 3) of analyzing the cell proliferation ability of hAD-MSCs after thawing, the hAD-MSCs being cryopreserved for 31 days in 4 liquids for cryopreserving a cell comprising 1.25 to 10% PG (LR containing 1.25% PG, 2.7% trehalose, and 4.5% dextran ["1.25% PG + Tre + D" in the figure]; LR containing 2.5% PG, 2.7% trehalose, and 4.5% dextran ["2.5% PG + Tre + D" in the figure]; LR containing 5% PG, 2.7% trehalose, and 4.5% dextran ["5% PG + Tre + D" in the figure]; and LR containing 10% PG, 2.7% trehalose, and 4.5% dextran ["10% PG + Tre + D" in the figure]) . "*" and "**" in the figure show that there is a statistically significant difference (p < 0.05 and p < 0.01, respectively) from "10% PG."

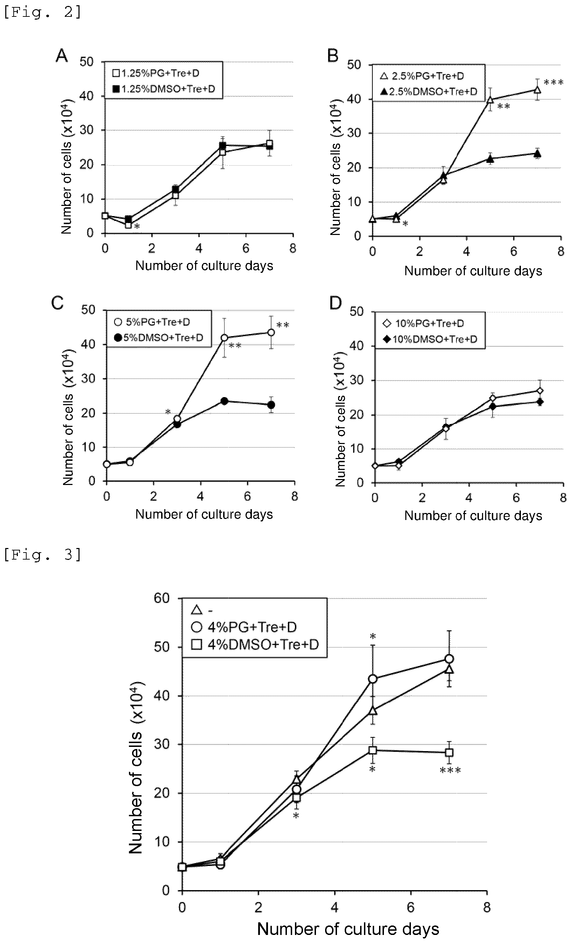

[Figure 2] Figure 2 is diagrams showing results of comparing the liquids for cryopreserving a cell having DMSO and PG having the same concentrations based on the results of Figure 1. "**" and "***" in the figure show that there is a statistically significant difference (p < 0.01 and p < 0.001, respectively) between samples having the same number of culture days.
[Figure 3] Figure 3 is a diagram showing results (mean value ± SD, n = 6) of analyzing the cell proliferation ability of hAD-MSCs after thawing, the hAD-MSCs being cryopreserved for 23 days in trehalose and dextran-containing liquids for cryopreserving a cell comprising 4% PG or 4% DMSO (LR containing 4% PG, 2.88% trehalose, and 4.8% dextran ["4% PG + Tre + D" in the figure]; and LR containing 4% DMSO, 2.88% trehalose, and 4.8% dextran ["4% DMSO + Tre + D" in the figure]), and the cell proliferation ability of hAD-MSCs not cryopreserved ("-" in the figure). "*" and "***" in the figure indicate that there is a statistically significant difference (p < 0.05 and p < 0.001, respectively) from "-" having the same number of culture days.
[Figure 4] Figure 4 is diagrams showing results (mean value ± SD, n = 3) of analyzing the cell proliferation ability of hAD-MSCs after thawing, the hAD-MSCs being cryopreserved for 44 days (Figures 4A and 4B) or 29 days (Figures 4C and 4D) in 4 base liquids comprising 1.25 to 10% PG (LR [Figure 4A]; LR containing dextran [Figure 4B]; LR containing trehalose [Figure 4C]; and LR containing trehalose and dextran [Figure 4D]). "*," "**," and "***" in the figure show that there is a statistically significant difference (p < 0.05, p < 0.01, and p < 0.001, respectively) between samples having the same number of culture days.

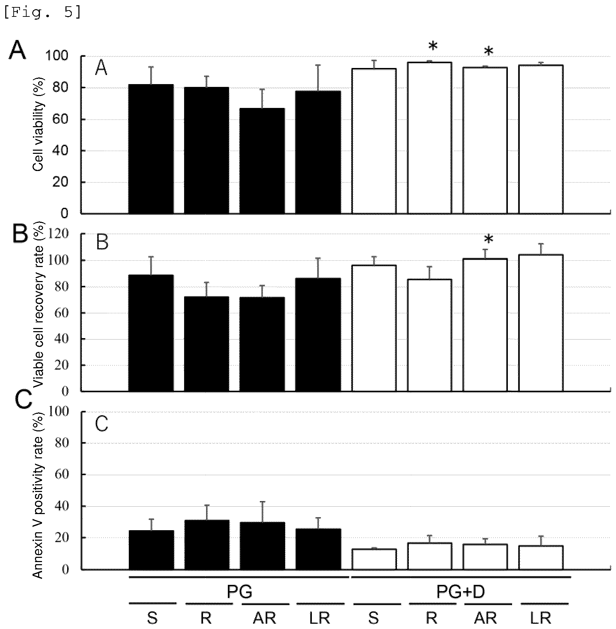

[Figure 5] Figure 5 is diagrams showing results (mean value ± SD, n = 3) of analyzing the cell viability (Figure 5A), the viable cell recovery rate (Figure 5B), and the annexin V positivity rate (Figure 5C) of hAD-MSCs immediately after thawing, the hAD-MSCs being cryopreserved for 33 days in 4 base liquids comprising 5% PG (physiological saline [PG/S], Ringer's solution [PG/R], acetated Ringer's solution [PG/AR], and lactated Ringer's solution [PG/LR]) (each black bar graph in the figure) or the 4 base liquids comprising 5% PG and 4.75% dextran ("PG + D/S, " "PG + D/R," "PG + D/AR, " and "PG + D/LR, " respectively) . "*" in the figure shows that there is a statistically significant difference (p < 0.05) from the corresponding "PG."

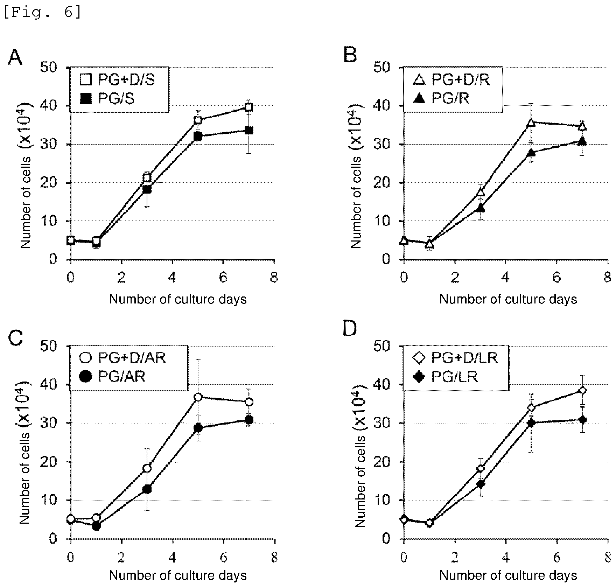

[Figure 6] Figure 6A is a diagram showing results (mean value ± SD, n = 3) of analyzing the cell proliferation ability of hAD-MSCs after thawing, the hAD-MSCs being cryopreserved for 70 days in physiological saline comprising 5% PG ("PG/S" in the figure) or physiological saline comprising 5% PG and 4.75% dextran ("PG + D/S" in the figure) . Figure 6B is a diagram showing results (mean value ± SD, n = 3) of analyzing the cell proliferation ability of hAD-MSCs after thawing, the hAD-MSCs being cryopreserved for 70 days in Ringer's solution comprising 5% PG ("PG/R" in the figure) or Ringer's solution comprising 5% PG and 4.75% dextran ("PG + D/R" in the figure). Figure 6C is a diagram showing results (mean value ± SD, n = 3) of analyzing the cell proliferation ability of hAD-MSCs after thawing, the hAD-MSCs being cryopreserved for 70 days in acetated Ringer's solution comprising 5% PG ("PG/AR" in the figure) or acetated Ringer's solution comprising 5% PG and 4.75% dextran ("PG + D/AR" in the figure). Figure 6D is a diagram showing results (mean value ± SD, n = 3) of analyzing the cell proliferation ability of hAD-MSCs after thawing, the hAD-MSCs being cryopreserved for 70 days in lactated Ringer's solution comprising 5% PG ("PG/LR" in the figure) or lactated Ringer's solution comprising 5% PG and 4.75% dextran ("PG + D/LR" in the figure).
[Figure 7] Figure 7 is diagrams showing results (mean value ± SD, n = 4) of analyzing the cell viability (Figure 7A), the viable cell recovery rate (Figure 7B), and the annexin V positivity rate (Figure 7C) of hAD-MSCs immediately after thawing, the hAD-MSCs being cryopreserved for 40 days in liquids for cryopreserving a cell comprising 1.25 to 10% PG and dextran, that is, 8 liquids for cryopreserving a cell (LR containing 1.25% PG and 4.94% dextran (["1.25%" in the figure]; LR containing 2.5% PG and 4.88% dextran ["2.5%" in the figure]; LR containing 3.75% PG and 4.81% dextran ["3.75%" in the figure]; LR containing 5% PG and 4.75% dextran ["5%" in the figure]; LR containing 6.25% PG and 4.69% dextran ["6.25%" in the figure]; LR containing 7.5% PG and 4.63% dextran ["7.5%" in the figure]; LR containing 8.75% PG and 4.56% dextran ["8.75%" in the figure]; and LR containing 10% PG and 4.5% dextran ["10%" in the figure]). "*, " "**, " and "***" in the figure show that there is a statistically significant difference (p < 0.05, p < 0.01, and p < 0.001, respectively) from "10%."
[Figure 8] Figure 8 is a diagram showing results (mean value ± SD, n = 3) of analyzing the cell proliferation ability of hAD-MSCs after thawing, the hAD-MSCs being cryopreserved for 28 days in the 8 liquids for cryopreserving a cell in Figure 7. "*" and "**" in the figure show that there is a statistically significant difference (p < 0.05 and p < 0.01, respectively) from "LR containing 10% PG and 4.5% dextran."

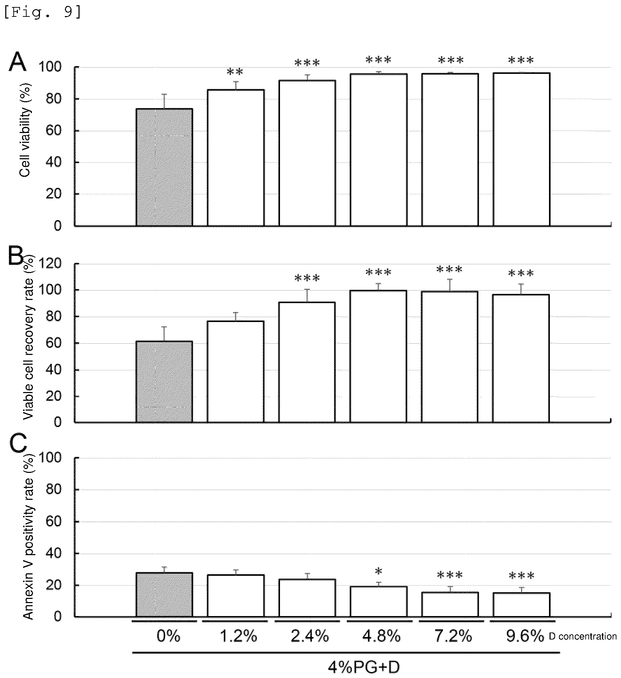

[Figure 9] Figure 9 is diagrams showing results (mean value ± SD, n = 4) of analyzing the cell viability (Figure 9A), the viable cell recovery rate (Figure 9B), and the annexin V positivity rate (Figure 9C) of hAD-MSCs immediately after thawing, the hAD-MSCs being cryopreserved for 38 days in liquids for cryopreserving a cell comprising 4% PG and 0 to 9.6% dextran, that is, 6 liquids for cryopreserving a cell (LR containing 4% PG ["0%" in the figure]; LR containing 4% PG and 1.2% dextran ["1.2%" in the figure]; LR containing 4% PG and 2.4% dextran ["2.4%" in the figure]; LR containing 4% PG and 4.8% dextran ["4.8%" in the figure]; LR containing 4% PG and 7.2% dextran ["7.2%" in the figure]; and LR containing 4% PG and 9.6% dextran ["9.6%" in the figure]). "*," "**," and "***" in the figure show that there is a statistically significant difference (p < 0.05, p < 0.01, and p < 0.001, respectively) from "0%."

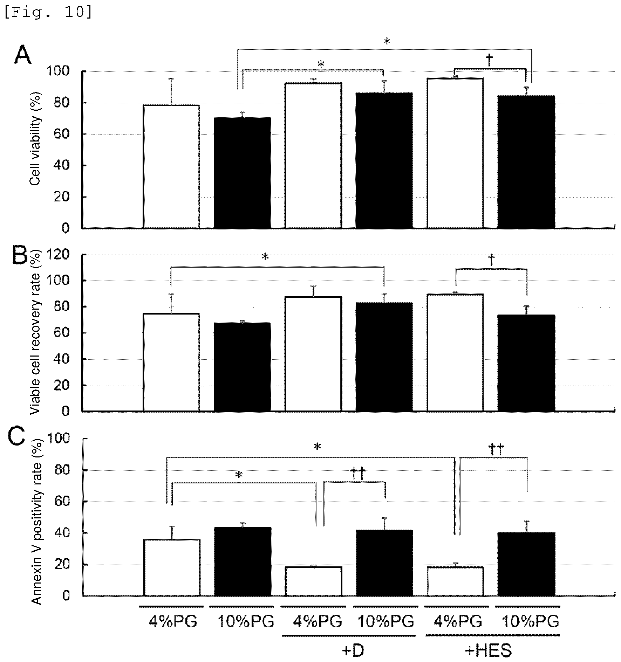

[Figure 10] Figure 10 is diagrams showing results (mean value ± SD, n = 3) of analyzing the cell viability (Figure 10A), the viable cell recovery rate (Figure 10B), and the annexin V positivity rate (Figure 10C) of hAD-MSCs immediately after thawing, the hAD-MSCs being cryopreserved for 24 days in 6 liquids for cryopreserving a cell (LR containing 4% PG ["4% PG" in the figure]; LR containing 10% PG ["10% PG" in the figure]; LR containing 4% PG and 4.8% dextran ["4% PG + D" in the figure]; LR containing 10% PG and 4.5% dextran ["10% PG + D" in the figure]; LR containing 4% PG and 4.8% HES ["4% PG + HES" in the figure]; and LR containing 10% PG and 4.5% HES ["10% PG + HES" in the figure]). "*" in the figure shows that there is a statistically significant difference (p < 0.05) by Dunnett's test, and "†" and "††" in the figure show that there is a statistically significant difference (p < 0.05 and p < 0.01, respectively) by Student's t test.

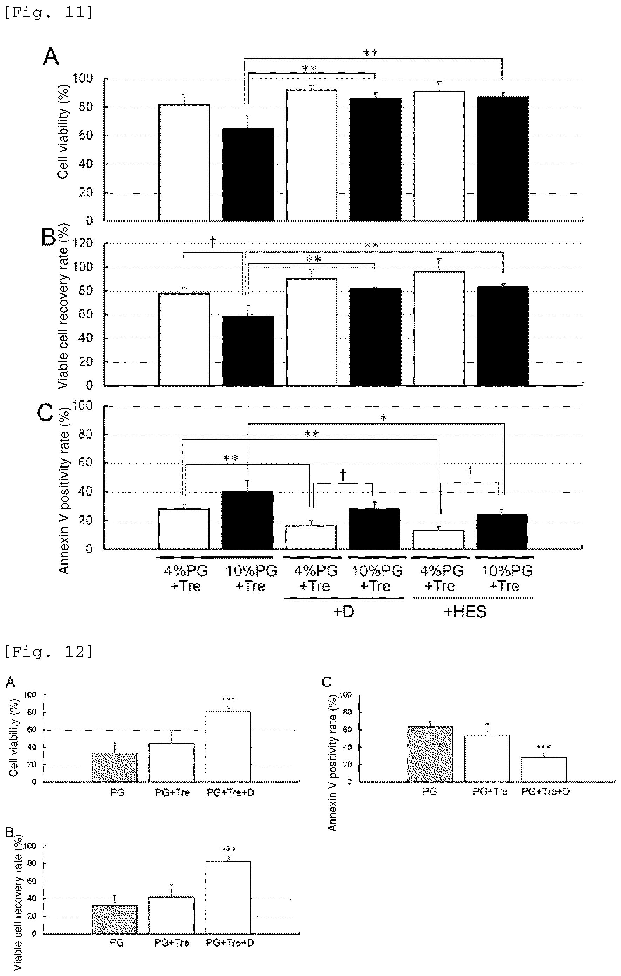

[Figure 11] Figure 11 is diagrams showing results (mean value ± SD, n = 3) of analyzing the cell viability (Figure 11A), the viable cell recovery rate (Figure 11B), and the annexin V positivity rate (Figure 11C) of hAD-MSCs immediately after thawing, the hAD-MSCs being cryopreserved for 24 days in 6 liquids for cryopreserving a cell (LR containing 4% PG and 2.88% trehalose ["4% PG + Tre" in the figure]; LR containing 10% PG and 2.7% trehalose ["10% PG + Tre" in the figure]; LR containing 4% PG, 2.88% trehalose, and 4.8% dextran ["4% PG + Tre + D" in the figure]; LR containing 10% PG, 2.7% trehalose, and 4.5% dextran ["10% PG + Tre + D" in the figure]; LR containing 4% PG, 2.88% trehalose, and 4.8% HES ["4% PG + Tre + HES" in the figure]; and LR containing 10% PG, 2.7% trehalose, and 4.5% HES ["10% PG + Tre + HES" in the figure]). "*" and "**" in the figure show that there is a statistically significant difference (p < 0.05 and p < 0.01, respectively) by Dunnett's test, and "†" in the figure shows that there is a statistically significant difference (p < 0.05) by Student's t test.
[Figure 12] Figure 12 is diagrams showing results (mean value ± standard deviation [SD], n = 4) of analyzing the cell viability (Figure 12A), the viable cell recovery rate (Figure 12B), and the annexin V positivity rate (Figure 12C) of human adipose-derived mesenchymal stem cells (hAD-MSCs) immediately after thawing, the hAD-MSCs being cryopreserved for 28 days in 3 liquids for cryopreserving a cell (lactated Ringer's solution containing 2.5% PG ["PG" in the figure]; lactated Ringer's solution containing 2.5% PG and 2.925% trehalose ["PG + Tre" in the figure]; and lactated Ringer's solution containing 2.5% PG, 2.925% trehalose, and 4.875% dextran ["PG + Tre + D" in the figure]). "*" and "***" in the figure show that there is a statistically significant difference (p < 0.05 and p < 0.001, respectively) from "PG."

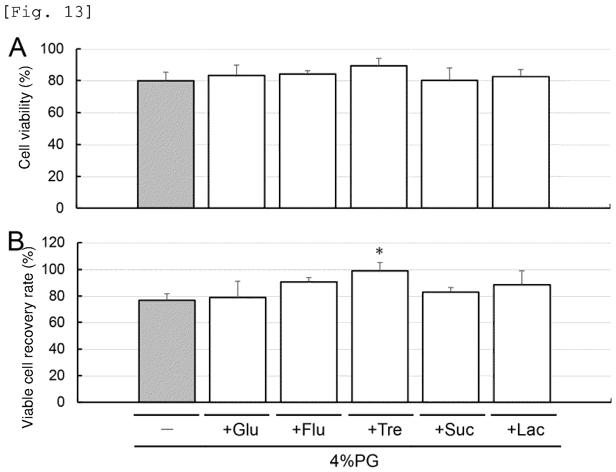

[Figure 13] Figure 13 is diagrams showing results (mean value ± SD, n = 4) of analyzing the cell viability (Figure 13A) and the viable cell recovery rate (Figure 13B) of hAD-MSCs immediately after thawing, the hAD-MSCs being cryopreserved for 35 days in liquids for cryopreserving a cell containing 4% PG comprising 5 sugars (glucose, fructose, trehalose, sucrose, or lactose) ("+Glu," "+Flu," "+Tre," "+Suc," and "+Lac," respectively, in the figure) or a liquid for cryopreserving a cell containing 4% PG without these sugars ("-" in the figure) . "*" in the figure shows that there is a statistically significant difference (p < 0.05) from " -."

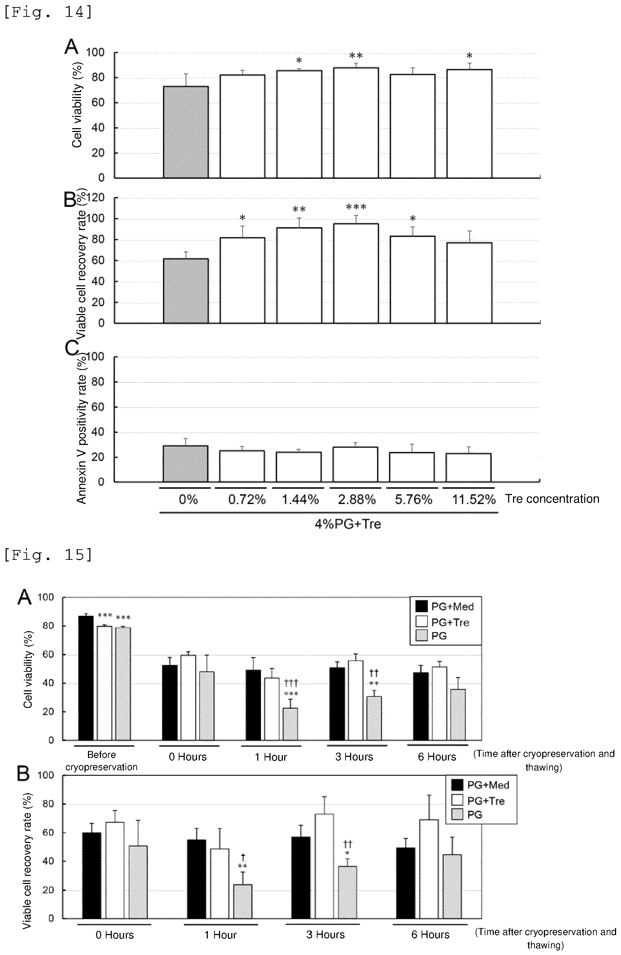

[Figure 14] Figure 14 is diagrams showing results (mean value ± SD, n = 4) of analyzing the cell viability (Figure 14A), the viable cell recovery rate (Figure 14B), and the annexin V positivity rate (Figure 14C) of hAD-MSCs immediately after thawing, the hAD-MSCs being cryopreserved for 32 days in liquids for cryopreserving a cell containing 4% PG and 0 to 11.52% trehalose, that is, 6 liquids for cryopreserving a cell (LR containing 4% PG ["0%" in the figure]; LR containing 4% PG and 0.72% trehalose ["0.72%" in the figure]; LR containing 4% PG and 1.44% trehalose ["1.44%" in the figure]; LR containing 4% PG and 2.88% trehalose ["2.88%" in the figure]; LR containing 4% PG and 5.76% trehalose ["5.76%" in the figure]; and LR containing 4% PG and 11.52% trehalose ["11.52%" in the figure]). "*, " "**," and "***" in the figure show that there is a statistically significant difference (p < 0.05, p < 0.01, and p < 0.001, respectively) from "0%."
[Figure 15] Figure 15 is diagrams showing results (mean value ± SD, n = 4) of analyzing the cell viability (Figure 15A) of hCD8-positive T cells before cryopreservation, the hCD8-positive T cells being suspended in 3 base liquids comprising 4% PG (culture medium containing serum ["PG + Med" in the figure], LR containing trehalose ["PG + Tre" in the figure], and LR ["PG" in the figure]), and the cell viability (Figure 15A) and the viable cell recovery rate (Figure 15B) of the hCD8-positive T cells immediately (0 hours) after thawing and 1 hour, 3 hours, and 6 hours after thawing, the hCD8-positive T cells being cryopreserved for 46 to 167 days. "*," "**," and "***" in the figure show that there is a statistically significant difference (p < 0.05, p < 0.01, and p < 0.001, respectively) from "PG + Med," and "†," "††," and "†††" in the figure indicate that there is a statistically significant difference (p < 0.05, p < 0.01, and p < 0.001, respectively) in the two-group comparison between "PG + Tre" and "PG."

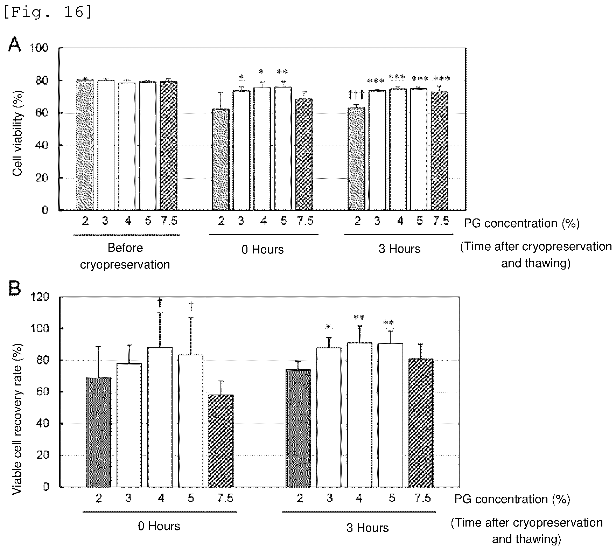

[Figure 16] Figure 16 is diagrams showing results (mean value ± SD, n = 4) of analyzing the cell viability (Figure 16A) of hCD8-positive T cells before cryopreservation, the hCD8-positive T cells being suspended in liquids for cryopreserving a cell containing 2 to 7.5% PG, that is, 5 liquids for cryopreserving a cell (LR containing 2% PG, 2.7% trehalose, and 4.5% dextran ["2" in the figure]; LR containing 3% PG, 2.7% trehalose, and 4.5% dextran ["3" in the figure]; LR containing 4% PG, 2.7% trehalose, and 4.5% dextran ["4" in the figure]; LR containing 5% PG, 2.7% trehalose, and 4.5% dextran ["5" in the figure]; and LR containing 7.5% PG, 2.7% trehalose, and 4.5% dextran ["7.5" in the figure]), and the cell viability (Figure 16A) and the viable cell recovery rate (Figure 16B) of the hCD8-positive T cells immediately (0 hours) after thawing and 3 hours after thawing, the hCD8-positive T cells being cryopreserved for 54 to 74 days. "*," "**," and "***" in the figure show that there is a statistically significant difference (p < 0.05, p < 0.01, and p < 0.001, respectively) from "2," and "†" and "†††" in the figure show that there is a statistically significant difference (p < 0.05 and p < 0.001, respectively) from "7.5."

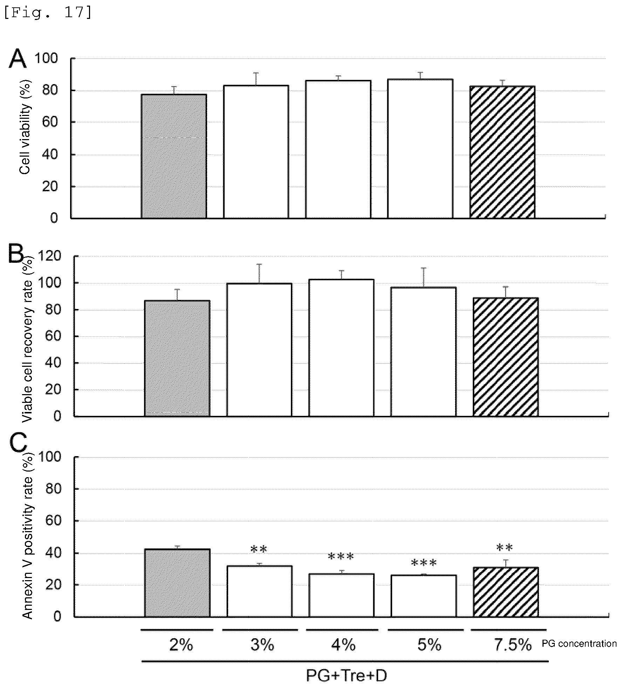

[Figure 17] Figure 17 is diagrams showing results (mean value ± SD, n = 3) of analyzing the cell viability (Figure 17A), the viable cell recovery rate (Figure 17B), and the annexin V positivity rate (Figure 17C) of hCD8-positive T cells immediately after thawing, the hCD8-positive T cells being cryopreserved for 46 days in the liquids for cryopreserving a cell containing 2 to 7.5% PG used in Figure 16. "**" and "***" in the figure show that there is a statistically significant difference (p < 0.01 and p < 0.001, respectively) from "2%."

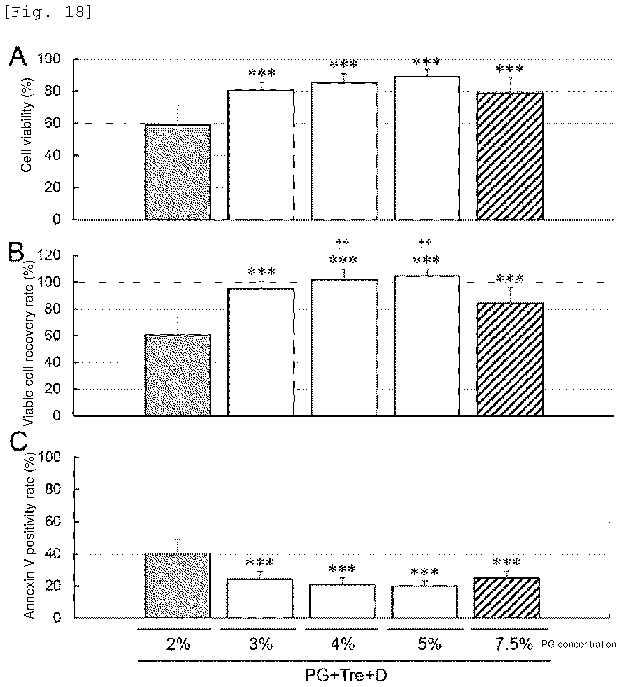

[Figure 18] Figure 18 is diagrams showing results (mean value ± SD, n = 6) of analyzing the cell viability (Figure 18A), the viable cell recovery rate (Figure 18B), and the annexin V positivity rate (Figure 18C) of hCD4-positive T cells immediately after thawing, the hCD4-positive T cells being cryopreserved for 40 to 44 days in the liquids for cryopreserving a cell containing 2 to 7.5% PG used in Figure 16. "***" in the figure shows that there is a statistically significant difference (p < 0.001) from "2%." "††" in the figure shows that there is a statistically significant difference (p < 0.01) from "7.5%."

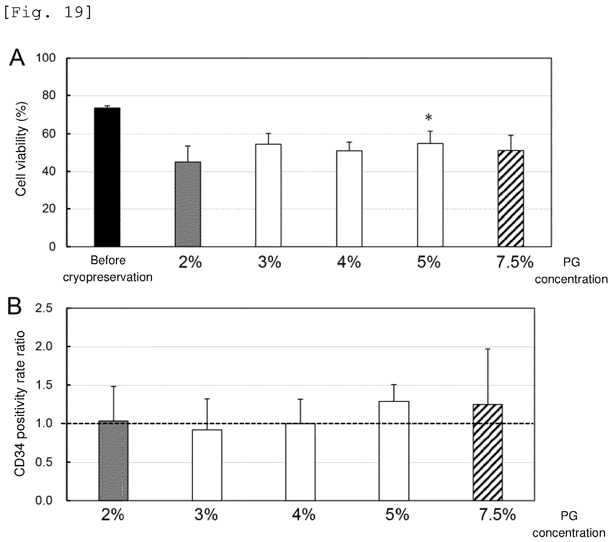

[Figure 19] Figure 19 is diagrams showing results (mean value ± SD, n = 4) of the cell viability (Figure 19A) and the CD34 positivity rate ratio (ratio of CD34 positivity rate immediately after freezing and thawing to that before cryopreservation) (Figure 19B) resulting from analyzing the cell viability and the CD34 positivity rate of hCD34-positive hematopoietic stem cells before cryopreservation, and analyzing the cell viability and the CD34 positivity rate of the hCD34-positive hematopoietic stem cells immediately after thawing, the hCD34-positive hematopoietic stem cells being cryopreserved for 14 days in the liquids for cryopreserving a cell containing 2 to 7.5% PG used in Figure 16. "*" in the figure shows that there is a statistically significant difference (p < 0.05) from "2%."

### Mode of Carrying Out the Invention

The liquid for cryopreserving a mammalian cell according to the present invention is a liquid comprising 2.5 to 8.75 (v/v)% propylene glycol (that is, the present cryopreservation liquid), which is specified to the application of "in order to cryopreserve a mammalian cell." The present cryopreservation liquid, in which the concentration of propylene glycol is 2.5 to 5.0 (v/v)%, can more effectively increase the proliferation ability of a mammalian cell after freezing and thawing than when a mammalian cell is cryopreserved in a liquid for cryopreserving a cell comprising propylene glycol having a concentration of other than 2.5 to 5.0 (v/v)% or in a liquid for cryopreserving a cell comprising DMSO. Because of this, the present cryopreservation liquid, in which the concentration of propylene glycol is 2.5 to 5.0 (v/v)%, may be specified to the application of "in order to increase the proliferation ability of a mammalian cell after freezing and thawing."

In addition, the present cryopreservation liquid (liquid comprising 2.5 to 8.75 (v/v)% propylene glycol) can keep the cell viability at a higher value when preserved at room temperature (1°C to 30°C) after thawing than when a mammalian cell is cryopreserved in a liquid for cryopreserving a cell comprising DMSO or in a liquid for cryopreserving a cell comprising propylene glycol having a concentration of other than 2.5 to 8.75 (v/v)%. Because of this, the present cryopreservation liquid may be specified to the application of "in order to increase the cell viability of a mammalian cell after freezing and thawing."

The method for cryopreserving a mammalian cell according to the present invention is not particularly limited as long as the method is a method comprising a step (a) of cryopreserving the present cryopreservation liquid containing a mammalian cell (in other words, cryopreserving a mammalian cell in the present cryopreservation liquid) (that is, the present cryopreservation method), and in such a step (a), a mammalian cell may be frozen by using a slow freezing method and then preserved, or may be frozen by using a quick freezing method (vitrification method) and then preserved. Examples of such a slow freezing method include a method involving transferring the present cryopreservation liquid containing a mammalian cell to a tube or a vial for cell preservation and freezing the same in a low-temperature freezer or an ultra-low temperature freezer (usually within the range of -20°C to -150°C) and then preserving the same in liquid nitrogen (usually within the range of -150°C to -196°C). Examples of the above quick freezing method include a method involving suspending a mammalian cell in the present cryopreservation liquid, then if necessary, transferring the resulting suspension into a straw, quickly freezing the same in liquid nitrogen (usually within the range of -150°C to -196°C), and preserving the same.

Herein, the concentration of propylene glycol may be within the range of 2.5 to 8.75 (v/v)%, and examples thereof include
2.5 to 7.5%, 2.5 to 6.25%, 2.5 to 5.0%, 2.5 to 4.9%, 2.5 to 4.8%, 2.5 to 4.6%, 2.5 to 4.4%, 2.5 to 4.2%, 2.5 to 4.0%, 2.5 to 3.8%, 2.5 to 3.6%, 2.5 to 3.4%, 2.5 to 3.2%, 2.5 to 3.0%, 2.5 to 2.8%, 2.5 to 2.6%, 2.6 to 8.75%, 2.8 to 8.75%, 3.0 to 8.75%, 3.2 to 8.75%, 3.4 to 8.75%, 3.6 to 8.75%, 3.8 to 8.75%, 4.0 to 8.75%, 4.2 to 8.75%, 4.4 to 8.75%, 4.6 to 8.75%, 4.8 to 8.75%, 5.0 to 8.75%, 6.25 to 8.75%, 7.5 to 8.75%, 2.6 to 7.5%, 2.6 to 6.25%, 2.6 to 5.0%, 2.6 to 4.9%, 2.6 to 4.8%, 2.6 to 4.6%, 2.6 to 4.4%, 2.6 to 4.2%, 2.6 to 4.0%, 2.6 to 3.8%, 2.6 to 3.6%, 2.6 to 3.4%, 2.6 to 3.2%, 2.6 to 3.0%, 2.6 to 2.8%, 2.8 to 7.5%, 2.8 to 6.25%, 2.8 to 5.0%, 2.8 to 4.9%, 2.8 to 4.8%, 2.8 to 4.6%, 2.8 to 4.4%, 2.8 to 4.2%, 2.8 to 4.0%, 2.8 to 3.8%, 2.8 to 3.6%, 2.8 to 3.4%, 2.8 to 3.2%, 2.8 to 3.0%, 3.0 to 7.5%, 3.0 to 6.25%, 3.0 to 5.0%, 3.0 to 4.9%, 3.0 to 4.8%, 3.0 to 4.6%, 3.0 to 4.4%, 3.0 to 4.2%, 3.0 to 4.0%, 3.0 to 3.8%, 3.0 to 3.6%, 3.0 to 3.4%, 3.0 to 3.2%, 3.2 to 7.5%, 3.2 to 6.25%, 3.2 to 5.0%, 3.2 to 4.9%, 3.2 to 4.8 %, 3.2 to 4.6%, 3.2 to 4.4%, 3.2 to 4.2%, 3.2 to 4.0%, 3.2 to 3.8%, 3.2 to 3.6%, 3.2 to 3.4%, 3.4 to 7.5%, 3.4 to 6.25%, 3.4 to 5.0%, 3.4 to 4.9%, 3.4 to 4.8%, 3.4 to 4.6%, 3.4 to 4.4%, 3.4 to 4.2%, 3.4 to 4.0%, 3.4 to 3.8%, 3.4 to 3.6%, 3.6 to 7.5%, 3.6 to 6.25%, 3.6 to 5.0%, 3.6 to 4.9%, 3.6 to 4.8%, 3.6 to 4.6%, 3.6 to 4.4%, 3.6 to 4.2%, 3.6 to 4.0%, 3.6 to 3.8%, 3.8 to 7.5%, 3.8 to 6.25%, 3.8 to 5.0%, 3.8 to 4.9%, 3.8 to 4.8%, 3.8 to 4.6%, 3.8 to 4.4%, 3.8 to 4.2%, 3.8 to 4.0%, 4.0 to 7.5%, 4.0 to 6.25%, 4.0 to 5.0%, 4.0 to 4.9%, 4.0 to 4.8%, 4.0 to 4.6%, 4.0 to 4.4%, 4.0 to 4.2%, 4.2 to 7.5%, 4.2 to 6.25%, 4.2 to 5.0%, 4.2 to 4.9%, 4.2 to 4.8 %, 4.2 to 4.6%, 4.2 to 4.4%, 4.4 to 7.5%, 4.4 to 6.25%, 4.4 to 5.0%, 4.4 to 4.9%, 4.4 to 4.8%, 4.4 to 4.6%, 4.6 to 7.5%, 4.6 to 6.25%, 4.6 to 5.0%, 4.6 to 4.9%, 4.6 to 4.8%, 4.8 to 7.5%, 4.8 to 6.25%, 4.8 to 5.0%, 4.8 to 4.9%, 4.9 to 7.5%, 4.9 to 6.25%, 4.9 to 5.0%, 5.0 to 7.5%, 5.0 to 6.25%, and 6.25 to 7.5%.

The present cryopreservation liquid preferably further comprises a polymer compound. As used herein, the polymer compound means a compound having a weight average molecular weight (Mw) of 1 × 10⁴ or more. Examples of such a polymer compound include a polymer compound selected from dextran or a derivative thereof or a salt of the dextran or the derivative (hereinafter sometimes referred to as "dextran group"); hydroxyethyl starch (also referred to as hydroxyethylated starch) or a derivative thereof or a salt of the hydroxyethyl starch or the derivative (hereinafter sometimes referred to as "hydroxyethyl starch group"); albumin; carboxymethylcellulose or a salt thereof; xanthan gum or a salt thereof; gelatin; amylopectin or a salt thereof; and the like, and among these, a suitable example thereof is a polymer compound selected from dextran group and hydroxyethyl starch group, because the effect thereof has been demonstrated in the present Example described later. The present cryopreservation liquid may or may not comprise a polymer compound other than dextran group and hydroxyethyl starch group.

Dextran in the above dextran group is not particularly limited as long as it is a polysaccharide (C₆H₁₀O₅)ₙ consisting of D-glucose and has an α1→6 linkage in the main chain thereof, and the weight average molecular weight of dextran (Mw) can be, for example, within the range of 1 × 10⁴ to 5 × 10⁵ (for example, dextran 40 [Mw = 40000], dextran 70 [Mw = 70000] ) . Such dextran can be produced by any known method such as chemical synthesis, microbial production, or enzymatic production, but a commercially available product can also be used. Examples of the commercially available product include Dextran 40 (manufactured by Tokyo Chemical Industry Co., Ltd.) and Dextran 70 (manufactured by Tokyo Chemical Industry Co., Ltd.).

Examples of the dextran derivative among the above dextran group include carboxylated dextran and diethylaminoethyl (DEAE)-dextran.

Hydroxyethyl starch in the above hydroxyethyl starche group is not particularly limited as long as it is a mixture of amylose in which α-D-glucose is linearly linked (α-1,4 linkage) and amylopectin having a branch (α-1,6 linkage) and is a substance in which one or more of C2, C3, and C6 of the glucose unit are hydroxyethylated (derivative of amylopectin); and the Mw of hydroxyethyl starch can be, for example, within the range of 5 × 10⁴ to 5 × 10⁶ (for example, 7 × 10⁴ or 2 × 10⁵). In addition, the degree of substitution (the number of hydroxyethyl groups per glucose unit) of the above hydroxyethyl starch is not particularly limited, and can be, for example, within the range of 0.4 to 0.8 (for example, 0.50 to 0.55). Such hydroxyethyl starch can be produced by any known method such as chemical synthesis, microbial production, or enzymatic production, but a commercially available product can also be used. Examples of the commercially available product include HES (manufactured by Fresenius Kabi Austria GmbH).

Examples of the hydroxyethyl starch derivative in theabove hydroxyethyl starche group include DEAE-hydroxyethyl starch.

The concentration of the polymer compound in the present cryopreservation liquid is not particularly limited as long as the effect of use thereof in combination with PG is recognized, and examples of the lower limit value of the concentration of the polymer compound include 1.0 (w/v)%, 1.2 (w/v)%, 1.4 (w/v)%, 1.6 (w/v)%, 1.8 (w/v)%, 2.0 (w/v)%, 2.2 (w/v)%, 2.4 (w/v)%, 2.6 (w/v)%, 2.8 (w/v)%, 3.0 (w/v)%, 3.2 (w/v)%, 3.4 (w/v)%, 3.6 (w/v)%, 3.8 (w/v)%, 4.0 (w/v)%, 4.2 (w/v)%, 4.4 (w/v)%, 4.6 (w/v)%, and 4.8 (w/v)%. In addition, examples of the upper limit value of the concentration of the polymer compound in the present cryopreservation liquid include 20 (w/v)%, 16 (w/v)%, 13 (w/v)%, 10 (w/v)%, 9.8 (w/v)%, and 9.6 (w/v)%.

Therefore, examples of the concentration of the above polymer compound include
1.0 to 20%, 1.2 to 20%, 1.4 to 20%, 1.6 to 20%, 1.8 to 20%, 2.0 to 20%, 2.2 to 20%, 2.4 to 20%, 2.6 to 20%, 2.8 to 20%, 3.0 to 20%, 3.2 to 20%, 3.4 to 20%, 3.6 to 20%, 3.8 to 20%, 4.0 to 20%, 4.2 to 20%, 4.4 to 20%, 4.6 to 20%, 4.8 to 20%, 1.0 to 16%, 1.2 to 16%, 1.4 to 16%, 1.6 to 16%, 1.8 to 16%, 2.0 to 16%, 2.2 to 16%, 2.4 to 16%, 2.6 to 16%, 2.8 to 16%, 3.0 to 16%, 3.2 to 16%, 3.4 to 16%, 3.6 to 16%, 3.8 to 16%, 4.0 to 16%, 4.2 to 16%, 4.4 to 16%, 4.6 to 16%, 4.8 to 16%, 1.0 to 13%, 1.2 to 13%, 1.4 to 13%, 1.6 to 13%, 1.8 to 13%, 2.0 to 13%, 2.2 to 13%, 2.4 to 13%, 2.6 to 13%, 2.8 to 13%, 3.0 to 13%, 3.2 to 13%, 3.4 to 13%, 3.6 to 13%, 3.8 to 13%, 4.0 to 13%, 4.2 to 13%, 4.4 to 13%, 4.6 to 13%, 4.8 to 13%, 1.0 to 10%, 1.2 to 10%, 1.4 to 10%, 1.6 to 10%, 1.8 to 10%, 2.0 to 10%, 2.2 to 10%, 2.4 to 10%, 2.6 to 10%, 2.8 to 10%, 3.0 to 10%, 3.2 to 10%, 3.4 to 10%, 3.6 to 10%, 3.8 to 10%, 4.0 to 10%, 4.2 to 10%, 4.4 to 10%, 4.6 to 10%, 4.8 to 10%, 1.0 to 9.8%, 1.2 to 9.8%, 1.4 to 9.8%, 1.6 to 9.8%, 1.8 to 9.8%, 2.0 to 9.8%, 2.2 to 9.8%, 2.4 to 9.8%, 2.6 to 9.8%, 2.8 to 9.8%, 3.0 to 9.8%, 3.2 to 9.8%, 3.4 to 9.8%, 3.6 to 9.8%, 3.8 to 9.8%, 4.0 to 9.8%, 4.2 to 9.8%, 4.4 to 9.8%, 4.6 to 9.8%, 4.8 to 9.8%, 1.0 to 9.6%, 1.2 to 9.6%, 1.4 to 9.6%, 1.6 to 9.6%, 1.8 to 9. 6%, 2.0 to 9.6, 2.2 to 9.6 %, 2.4 to 9.6%, 2.6 to 9.6%, 2.8 to 9.6%, 3.0 to 9.6%, 3.2 to 9.6%, 3.4 to 9.6%, 3.6 to 9.6%, 3.8 to 9.6%, 4.0 to 9.6%, 4.2 to 9.6%, 4.4 to 9.6%, 4.6 to 9.6%, and 4.8 to 9.6%, and when the polymer compound is a dextran group, 1.2 to 9.6% is preferable, 2.4 to 9.6% is more preferable, and 4.8 to 9.6% is further preferable.

Examples of the range of the weight ratio of propylene glycol to a polymer compound in the present cryopreservation liquid further comprising the polymer compound include 1:0.1 to 1:10, 1:0.1 to 1:8, 1:0.1 to 1:6, 1:0.1 to 1:4, 1:0.2 to 1:10, 1:0.2 to 1:8, 1:0.2 to 1:6, 1:0.2 to 1:4, 1:0.3 to 1:10, 1:0.3 to 1:8, 1:0.3 to 1:6, 1:0.3 to 1:4, 1:0.4 to 1:10, 1:0.4 to 1:8, 1:0.4 to 1:6, 1:0.4 to 1:4, 1:0.5 to 1:10, 1:0.5 to 1:8, 1:0.5 to 1:6, and 1:0.5 to 1:4, and when the polymer compound is a dextran group, 1:0.1 to 1:4 is preferable, 1:0.3 to 1:4 is more preferable, and 1:0.5 to 1:4 is further preferable.

The present cryopreservation liquid preferably further comprises trehalose or a derivative thereof or a salt of the trehalose or the derivative (hereinafter sometimes referred to as "trehalose group"). Examples of trehalose in the trehalose group include α,α-trehalose, which is a disaccharide in which two α-glucoses are linked together in a 1,1-glycosidic linkage, α,β-trehalose, which is a disaccharide in which α-glucose and β-glucose are linked together in a 1,1-glycosidic linkage, and β,β-trehalose, which is a disaccharide in which two β-glucoses are linked together in a 1,1-glycosidic linkage, and among these, α,α-trehalose is preferable. Such trehalose can be produced by any known method such as chemical synthesis, microbial production, or enzymatic production, but a commercially available product can also be used. Examples of the commercially available product include trehalose dihydrate (manufactured by FUJIFILM Wako Pure Chemical Corporation).

The trehalose derivative in the above trehalose group is not particularly limited as long as it is a glycosyltrehalose in which one or more sugar units are linked to the disaccharide trehalose, and examples of the glycosyltrehalose include glucosyltrehalose, maltosyltrehalose, and maltotriosyltrehalose.

The concentration of trehalose group in the present cryopreservation liquid is not particularly limited as long as the effect of use thereof in combination with PG is recognized, and examples of the lower limit value of the concentration of trehalose group include 0.7 (w/v)%, 0.8 (w/v)%, 0.9 (w/v)%, 1.0 (w/v)%, 1.1 (w/v)%, 1.2 (w/v)%, 1.3 (w/v)%, and 1.4 (w/v)%. In addition, examples of the upper limit value of the concentration of trehalose group in the present cryopreservation liquid include 40 (w/v)%, 35 (w/v)%, 30 (w/v)%, 25 (w/v)%, 20 (w/v)%, 15 (w/v)%, 12 (w/v)%, and 6.0 (w/v)%.

Therefore, examples of the concentration of above trehalose group include
0.7 to 40%, 0.8 to 40%, 0.9 to 40%, 1.0 to 40%, 1.1 to 40%, 1.2 to 40%, 1.3 to 40%, 1.4 to 40%, 0.7 to 35%, 0.8 to 35%, 0.9 to 35%, 1.0 to 35%, 1.1 to 35%, 1.2 to 35%, 1.3 to 35%, 1.4 to 35%, 0.7 to 30%, 0.8 to 30%, 0.9 to 30%, 1.0 to 30%, 1.1 to 30%, 1.2 to 30%, 1.3 to 30%, 1.4 to 30%, 0.7 to 25%, 0.8 to 25%, 0.9 to 25%, 1.0 to 25%, 1.1 to 25%, 1.2 to 25%, 1.3 to 25%, 1.4 to 25%, 0.7 to 20%, 0.8 to 20%, 0.9 to 20%, 1.0 to 20%, 1.1 to 20%, 1.2 to 20%, 1.3 to 20%, 1.4 to 20%, 0.7 to 15%, 0.8 to 15%, 0.9 to 15%, 1.0 to 15%, 1.1 to 15%, 1.2 to 15%, 1.3 to 15%, 1.4 to 15%, 0.7 to 12%, 0.8 to 12%, 0.9 to 12%, 1.0 to 12%, 1.1 to 12%, 1.2 to 12%, 1.3 to 12%, 1.4 to 12%, 0.7 to 6.0%, 0.8 to 6.0%, 0.9 to 6.0%, 1.0 to 6.0%, 1.1 to 6.0%, 1.2 to 6.0%, 1.3 to 6.0%, and 1.4 to 6.0%.

Examples of the range of the weight ratio of propylene glycol and trehalose group in the present cryopreservation liquid further comprising trehalose group include 1:0.08 to 1:16, 1:0.08 to 1:14, 1:0.08 to 1:12, 1:0.08 to 1:10, 1:0.08 to 1:8, 1:0.08 to 1:6, 1:0.08 to 1:4, 1:0.08 to 1:2.4, 1:0.1 to 1:16, 1:0.1 to 1:14, 1:0.1 to 1:12, 1:0.1 to 1:10, 1:0.1 to 1:8, 1:0.1 to 1:6, 1:0.1 to 1:4, 1:0.1 to 1:2.4, 1:0.12 to 1:16, 1:0.12 to 1:14, 1:0.12 to 1:12, 1:0.12 to 1:10, 1:0.12 to 1:8, 1:0.12 to 1:6, 1:0.12 to 1:4, 1:0.12 to 1:2.4, 0.14 to 1:16, 1:0.14 to 1:14, 1:0.14 to 1:12, 1:0.14 to 1:10, 1:0.14 to 1:8, 1:0.14 to 1:6, 1:0.14 to 1:4, 1:0.14 to 1:2.4, 0.16 to 1:16, 1:0.16 to 1:14, 1:0.16 to 1:12, 1:0.16 to 1:10, 1:0.16 to 1:8, 1:0.16 to 1:6, 1:0.16 to 1:4, and 1:0.16 to 1:2.4.

The present cryopreservation liquid is a liquid (for example, isotonic solution, hypotonic solution, or hypertonic solution) that comprises 2.5 to 8.75 (v/v)% propylene glycol and can cryopreserve a mammalian cell, and is preferably an isotonic solution comprising 2.5 to 8.75 (v/v)% propylene glycol. As used herein, the "isotonic solution" means a liquid that has almost the same osmotic pressure as that of a body fluid or a cell fluid, and specifically means a liquid that has an osmotic pressure within the range of 250 to 380 mOsm/L. In addition, as used herein, the "hypotonic solution" means a liquid that has an osmotic pressure lower than that of a body fluid or a cell fluid, and specifically means a liquid that has an osmotic pressure of less than 250 mOsm/L. Such a hypotonic solution is preferably a hypotonic solution that does not cause cell rupture (specifically, a liquid that has an osmotic pressure within the range of 100 to less than 250 mOsm/L). In addition, as used herein, the "hypertonic solution" means a liquid that has an osmotic pressure higher than that of a body fluid or a cell fluid, and specifically means that the osmotic pressure is more than 380 mOsm/L (preferably within the range of more than 380 mOsm/L to 1000 mOsm/L).

The above isotonic solution is not particularly limited as long as it is an isotonic solution having the salt concentration, the sugar concentration, or the like adjusted with a sodium ion, a potassium ion, a calcium ion, or the like such that the osmotic pressure is almost the same as that of a body fluid or a cell fluid, specific examples thereof include physiological saline, physiological saline having a buffering effect (for example, PBS, Tris Buffered Saline (TBS), or HEPES buffered saline), Ringer's solution, lactated Ringer's solution, acetate Ringer's solution, bicarbonated Ringer's solution, a 5% glucose aqueous solution, a basal medium for animal cell culture (for example, DMEM, EMEM, RPMI-1640, α-MEM, F-12, F-10, or M-199), and an isotonic agent (for example, glucose, D-sorbitol, D-mannitol, lactose, or sodium chloride), and among these, a suitable example thereof is an isotonic solution selected from lactated Ringer's solution, physiological saline, Ringer's solution, and acetated Ringer's solution because the effect thereof has been demonstrated in the present Example described later. The isotonic solution may be a commercially available product or a self-prepared product. Examples of the commercially available product include OTSUKA NORMAL SALINE (manufactured by Otsuka Pharmaceutical Factory, Inc.) (isotonic sodium chloride solution), Ringer's Solution "OTSUKA" (manufactured by Otsuka Pharmaceutical Factory, Inc.) (Ringer's solution), Lactec (registered trademark) Injection (manufactured by Otsuka Pharmaceutical Factory, Inc.) (lactated Ringer's solution), Veen (registered trademark)-F Injection (manufactured by Fuso Pharmaceutical Industries, Ltd.) (acetated Ringer's solution), OTSUKA GLUCOSE INJECTION 5% (manufactured by Otsuka Pharmaceutical Factory, Inc.) (5% glucose aqueous solution), and BICANATE (registered trademark) Injection (manufactured by Otsuka Pharmaceutical Factory, Inc.) (bicarbonated Ringer's solution).

The present cryopreservation liquid may comprise a cryoprotective component other than PG (for example, DMSO; glycerol; ethylene glycol; PEG; sericin; isomaltooligosaccharide; or serum derived from a human, a cow, or the like), and one comprising no cryoprotective component other than PG is preferable because PG alone can effectively increase the proliferation ability of a mammalian cell after freezing and thawing.

The present cryopreservation liquid may or may not comprise an oligosaccharide other than trehalose group (for example, a disaccharide such as sucrose, lactose, or maltose; a trisaccharide such as maltotriose, raffinose, or melezitose; or a tetrasaccharide such as acarbose or stachyose), and a monosaccharide (for example, glucose, fructose, or galactose).

The present cryopreservation liquid usually does not comprise a substance selected from taurine or a derivative thereof or a salt thereof; and glycine or a derivative thereof or a salt thereof. Examples of such a taurine derivative include N-substituted taurine (for example, N-(2-acetamido)-2-aminoethanesulfonic acid, N,N-bis(2-hydroxyethyl)-2-aminoethanesulfonic acid, N-cyclohexyl-2-aminoethanesulfonic acid, 2-[4-(2-hydroxyethyl)-1-piperazinyl]ethanesulfonic acid, 2-morpholinoethanesulfonic acid, piperazine-1,4-bis(2-ethanesulfonic acid), or N-tris(hydroxymethyl)methyl-2-aminoethanesulfonic acid), hypotaurine, and thiotaurine. Examples of the above glycine derivative include sarcosine (N-methylglycine), N-ethylglycine, N-propylglycine, N,N-diethylglycine, N,N-dimethylglycine, N-amidinoglycine, N-amidino-N-methylglycine, glycylglycine, phenaceturic acid, glycine methyl ester, glycine ethyl ester, glycine n-butyl ester, glycine t-butyl ester, glycine n-propyl ester, glycine n-pentyl ester, and glycine benzyl ester. Examples of the "salts" of taurine, a taurine derivative, glycine, and a glycine derivative include an acid addition salt such as a hydrochloride, a hydrobromide, a hydroiodide, a phosphate, a nitrate, a sulfate, an acetate, a propionate, a toluenesulfonate, a succinate, an oxalate, a lactate, a tartrate, a glycolate, a methanesulfonate, a butyrate, a valerate, a citrate, a fumarate, a maleate, or a malate, a metal salt such as a sodium salt, a potassium salt, or a calcium salt, an ammonium salt, and an alkylammonium salt.

Herein, examples of the "salts" of (dextran, a dextran derivative, hydroxyethyl starch, a hydroxyethyl starch derivative, trehalose, a trehalose derivative, carboxymethylcellulose, xanthan gum, and amylopectin) include an acid addition salt such as a hydrochloride, a hydrobromide, a hydroiodide, a phosphate, a nitrate, a sulfate, an acetate, a propionate, a toluenesulfonate, a succinate, an oxalate, a lactate, a tartrate, a glycolate, a methanesulfonate, a butyrate, a valerate, a citrate, a fumarate, a maleate, or a malate, a metal salt such as a sodium salt, a potassium salt, or a calcium salt, an ammonium salt, and an alkylammonium salt. These salts are used as a solution when used, and the action thereof is preferably as effective as when using dextran, a dextran derivative, hydroxyethyl starch, a hydroxyethyl starch derivative, trehalose, a trehalose derivative, carboxymethylcellulose, xanthan gum, and amylopectin. The "salts" of (dextran, a dextran derivative, hydroxyethyl starch, a hydroxyethyl starch derivative, trehalose, a trehalose derivative, carboxymethylcellulose, xanthan gum, and amylopectin) may form hydrates or solvates, and any one thereof can be used alone, or two or more thereof can be used in appropriate combinations.

Examples of an optional component in the present cryopreservation liquid include a vitamin (for example, choline chloride, pantothenic acid, folic acid, nicotinamide, pyridoxal hydrochloride, riboflavin, thiamine hydrochloride, ascorbic acid, biotin, or inositol), a chelating agent (for example, EDTA, EGTA, citric acid, or salicylate), an antibiotic (for example, penicillin or streptomycin), a protein, and an antioxidant (for example, polyphenol or quercetin). As used herein, the "optional component" means a component that may or may not be comprised.

Examples of a specific cell of the above mammalian cell include a stem cell (for example, a stem cell administered via a blood vessel in regenerative medicine or the like); a pancreatic islet cell (for example, a pancreatic islet cell administered intravenously to a type I diabetic patient); a lymphoid cell such as a T cell, a natural killer (NK) cell, or a B cell; an antigen presenting cell such as a monocyte, a macrophage, or a dendritic cell; and a granulocyte such as a neutrophil, an eosinophil, a basophil, or a mast cell, and a suitable example thereof is a T cell because the effect thereof has been demonstrated in the present Example described later. Examples of such a T cell include an alpha beta T cell, a gamma delta T cell, a CD8-positive T cell, a CD4-positive T cell, a tumor infiltrating T cell, a memory T cell, a naive T cell, and an NKT cell. A T cell can be collected from bone marrow, peripheral blood, umbilical cord blood, or the like of a mammal by a known general method.

Herein, examples of the "mammal" include a rodent such as a mouse, a rat, a hamster, or a guinea pig, a lagomorph such as a rabbit, an ungulate such as a pig, a cow, a goat, a horse, or a sheep, a carnivore such as a dog or a cat, and a primate such as a human, a monkey, a rhesus monkey, a cynomolgus monkey, a marmoset, an orangutan, or a chimpanzee, and among these, suitable examples thereof are a mouse, a pig, and a human.

The above "stem cell" means an immature cell that has a self-renewal ability and a differentiation/proliferation ability. Stem cells comprise a subpopulation such as a pluripotent stem cell, a multipotent stem cell, or an unipotent stem cell, depending on the differentiation ability thereof. The pluripotent stem cell means a cell that cannot become an individual by itself, but has the ability to differentiate into all tissues or cells that constitute a living body. The multipotent stem cell means a cell that has the ability to differentiate into a plurality of, but not all, tissues or cells. The unipotent stem cell means a cell that has the ability to differentiate into a specific tissue or cell.

Examples of the pluripotent stem cell include an embryonic stem cell (ES cell), an EG cell, and an iPS cell. An ES cell can be produced by culturing an inner cell mass on a feeder cell or in a medium comprising LIF. A method for producing an ES cell is disclosed in, for example, WO 96/22362, WO 02/101057, US5,843,780, US6,200,806, or US6,280,718. An EG cell can be produced by culturing a primordial germ cell in a medium comprising mSCF, LIF, and bFGF (Cell, 70: 841-847, 1992). An iPS cell can be produced by introducing a reprogramming factor such as Oct3/4, Sox2, and Klf4 (and even c-Myc or n-Myc if necessary) into a somatic cell (for example, a fibroblast or a skin cell) (Cell, 126: p.663-676, 2006; Nature, 448: p.313-317, 2007; Nat Biotechnol, 26; p,101-106, 2008; Cell 131: p.861-872, 2007; Science, 318: p.1917-1920, 2007; Cell Stem Cells 1: p.55-70, 2007; Nat Biotechnol, 25: p.1177-1181, 2007; Nature, 448: p.318-324, 2007; Cell Stem Cells 2: p.10-12, 2008; Nature451: p.141-146, 2008; Science, 318: p.1917-1920, 2007). In addition, a stem cell established by culturing an early embryo prepared by nuclear transplantation of a somatic cell nucleus is also preferable as a pluripotent stem cell (Nature, 385,810 (1997); Science, 280,1256 (1998); Nature Biotechnology, 17,456 (1999); Nature, 394,369 (1998); Nature Genetics, 22,127 (1999); Proc. Natl. Acad.Sci.USA,96,14984 (1999)), Rideout III et al. (Nature Genetics, 24,109 (2000)).

Examples of the multipotent stem cell include a mesenchymal stem cell that can differentiate into a cell such as an adipocyte, an osteocyte, or a chondrocyte; a hematopoietic stem cell that can differentiate into a blood cell such as a white blood cell, a red blood cell, or a platelet (for example, a hematopoietic stem cell positive for at least one cell surface marker selected from CD34, CD110, CD111, CD112, and CD117, preferably a CD34-positive hematopoietic stem cell); a neural stem cell that can differentiate into a cell such as a neuron, an astrocyte, or an oligodendrocyte; and a somatic stem cell such as a bone marrow stem cell or a germ stem cell, and suitable examples thereof are a mesenchymal stem cell and a hematopoietic stem cell because the effect thereof has been demonstrated in the present Example described later. A multipotent stem cell can be isolated from a living body by a method known per se. For example, a mesenchymal stem cell can be collected from bone marrow, adipose tissue, peripheral blood, umbilical cord blood, or the like of a mammal by a known general method. In addition, a human mesenchymal stem cell can be isolated by culture and passage of a hematopoietic stem cell or the like after bone marrow aspiration (Journal of Autoimmunity, 30(2008) 163-171). For example, a hematopoietic stem cell can be collected from bone marrow, peripheral blood, umbilical cord blood, or the like of a mammal by a known general method. A multipotent stem cell can also be obtained by culturing the above pluripotent stem cell under an appropriate induction condition. The mesenchymal stem cell is preferably a human adipose-derived mesenchymal stem cell. In addition, the hematopoietic stem cell is preferably a human-derived CD34-positive hematopoietic stem cell.

The present cryopreservation liquid may be an embodiment that does not comprise a mammalian cell or an embodiment that comprises a mammalian cell, and when the present cryopreservation method is carried out, the present cryopreservation liquid is preferably an embodiment that comprises a mammalian cell (that is, the present cryopreservation liquid containing a mammalian cell). The present cryopreservation liquid containing a mammalian cell can be prepared by any method, and examples thereof include 1) a method for preparing the present cryopreservation liquid containing a mammalian cell by separating a mammalian cell-containing liquid into the supernatant (liquid) and a precipitate (mammalian cell) by a centrifugation treatment, then removing the supernatant (liquid), and adding the present cryopreservation liquid to the precipitate (mammalian cell) and 2) a method for preparing the present cryopreservation liquid containing a mammalian cell by mixing a liquid with a mammalian cell-containing liquid, the liquid being adjusted such that the final concentration of a component comprised in the present cryopreservation liquid is a target concentration (for example, for PG, 2.5 to 8.75 (v/v)%) when mixed with the mammalian cell-containing liquid. The present cryopreservation liquid containing a mammalian cell is preferably the present cryopreservation liquid comprising umbilical cord blood. The present cryopreservation liquid comprising umbilical cord blood can be prepared by mixing umbilical cord blood (that is, a liquid comprising a mammalian cell such as a hematopoietic stem cell) and a liquid comprising PG at any ratio (for example, 10:1 to 1:10 [for example, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, or 1:10]) according to the above method in 2) .

An example of the mammalian cell to be preserved in the present cryopreservation liquid is an adherent cell. As used herein, the "adherent" cell means an anchorage-dependent cell that can survive, proliferate, and produce a substance by adhering to an anchorage. Examples of the adherent stem cell include a pluripotent stem cell, a mesenchymal stem cell, a neural stem cell, a bone marrow stem cell, and a germ stem cell. The adherent stem cell is preferably a mesenchymal stem cell.

The mammalian cell (population) to be preserved in the present cryopreservation liquid may be isolated from a living body or subcultured in vitro, and is preferably isolated or purified. As used herein, "isolated or purified" means that the operation of removing a component other than the target component is carried out. The purity of the isolated or purified mammalian cell (the proportion of the target cell such as the number of mammalian stem cells to the total number of cells) is usually 30% or more, preferably 50% or more, more preferably 70% or more, and further preferably 90% or more (for example, 100%).

The mammalian cell (population) to be preserved in the present cryopreservation liquid is preferably in the state of a single cell. As used herein, the "state of a single cell" means that cells do not gather together with other cells to form a mass (that is, a non-aggregated state). A mammalian cell in the state of a single cell can be prepared by enzymatically treating a mammalian cell cultured in vitro with trypsin/EDTA or the like, and then suspending the cell by a method well known in the art such as pipetting or tapping. The proportion of the mammalian cell in the state of a single cell comprised in mammalian cells is usually 70% or more, preferably 90% or more, more preferably 95% or more, and further preferably 99% or more (for example, 100%). The proportion of the mammalian cell in the state of a single cell can be determined by dispersing mammalian cells in PBS, observing these under a microscope, and examining a plurality of (for example, 1000) randomly selected cells for the presence or absence of aggregation.

The present cryopreservation method is preferably a method further comprising a step (b) of freezing and thawing the mammalian cell and culturing the same after the above step (a). Examples of the culture period include 1 day or more, 2 days or more, 3 days or more, 4 days or more, 5 days or more, 6 days or more, 7 days or more, and 8 days or more, and the culture period is preferably 5 days or more.

In the above step (b), the method for freezing and thawing the mammalian cell is not particularly limited, and a frozen container (a tube for cell preservation, a vial for cell preservation, a straw, or the like) comprising the present cryopreservation liquid containing the mammalian cell may be naturally thawed at normal temperature (for example, within the range of 10 to 28°C), and from the viewpoint of preventing refreezing, the container is preferably soaked in warm water (for example, water within the range of 30 to 40°C) for quick thawing.

In the above step (b), the mammalian cell after freezing and thawing is cultured in an appropriate container (a culture plate, a culture dish, a culture flask, or the like) in the presence of an appropriate culture medium. Examples of such a culture medium include a culture medium for animal cell culture (DMEM, EMEM, IMDM, RPMI1640, αMEM, F-12, F-10, M-199, AIM-V, or the like) comprising 0.1 to 30 (v/v)% serum (FBS, CS, or the like), and MyeloCult H5100 medium (manufactured by STEMCELL Technologies, ST-05150). In addition, examples of a serum-free culture medium (serum-free culture medium) include the above culture medium for animal cell culture supplemented with an appropriate amount (for example, 1% to 30%) of a serum substitute such as the above commercially available B27 Supplement (-Insulin), N2 Supplement, B27 Supplement, or Knockout Serum Replacement.

In the above step (b), the culture temperature of the mammalian cell after freezing and thawing is usually within the range of about 30 to 40°C, and is preferably 37°C. In addition, the CO₂ concentration during culture is usually within the range of about 1 to 10%, and is preferably about 5%. In addition, the humidity during culture is usually within the range of about 70 to 100%, and preferably within the range of about 95 to 100%. In addition, the O₂ concentration during culture may be a normal oxygen concentration (18 to 22% O₂) or a low oxygen concentration (0 to 10% O₂) .

Hereinafter, the present invention will be described more specifically with reference to an Example, but the technical scope of the present invention is not limited to these examples.

### Example

### 1. Method

### [Preparation of liquids for cryopreserving cell used in Study 1]

1) LR containing 10 (v/v)% DMSO, 2.7 (w/v)% trehalose, and 4.5 (w/v)% dextran was prepared by mixing Cellstor-S (manufactured by Otsuka Pharmaceutical Factory, Inc.) (that is, lactated Ringer's solution containing 3 (w/v) % trehalose and 5 (w/v)% dextran) and CultureSure (registered trademark) DMSO (manufactured by FUJIFILM Wako Pure Chemical Corporation) at a ratio of 9:1.
2) LR containing 5 (v/v) % DMSO, 2.7 (w/v) % trehalose, and 4.5 (w/v)% dextran; LR containing 2.5 (v/v)% DMSO, 2.7 (w/v)% trehalose, and 4.5 (w/v)% dextran; and LR containing 1.25 (v/v)% DMSO, 2.7 (w/v)% trehalose, and 4.5 (w/v)% dextran were prepared by diluting the above "LR containing 10% DMSO, 2.7 (w/v) % trehalose, and 4.5 (w/v)% dextran" with Cellstor-S at a 2-fold common ratio.
3) LR containing 10 (v/v)% propylene glycol (PG) (manufactured by Maruishi Pharmaceutical Co., Ltd.), 2.7 (w/v)% trehalose, and 4.5 (w/v)% dextran was prepared by mixing Cellstor-S and PG at a ratio of 9:1.
4) LR containing 5 (v/v)% PG, 2.7 (w/v)% trehalose, and 4.5 (w/v)% dextran; LR containing 2.5 (v/v)% PG, 2.7 (w/v)% trehalose, and 4.5 (w/v)% dextran; and LR containing 1.25 (v/v)% PG, 2.7 (w/v) % trehalose, and 4.5 (w/v) % dextran were prepared by diluting the above "LR containing 10 (v/v)% PG, 2.7 (w/v) % trehalose, and 4.5 (w/v)% dextran" with Cellstor-S at a 2-fold common ratio.

### [Preparation of liquids for cryopreserving cell used in Study 2]

1) LR containing 4 (v/v)% PG, 2.88 (w/v)% trehalose, and 4.8 (w/v)% dextran was prepared by mixing 0.4 mL of the above PG and 9.6 mL of Cellstor-S.
2) LR containing 4 (v/v)% DMSO, 2.88 (w/v)% trehalose, and 4.8 (w/v)% dextran was prepared by mixing 0.4 mL of the above DMSO and 9.6 mL of Cellstor-S.

### [Preparation of liquids for cryopreserving cell used in Study 3]

1) LR containing 10 (v/v)% PG was prepared by mixing Lactec Injection (manufactured by Otsuka Pharmaceutical Factory, Inc.) and the above PG at a ratio of 9:1.
2) LR containing 5(v/v)% PG, LR containing 2.5(v/v)% PG, and LR containing 1.25(v/v)% PG were prepared by diluting the above "LR containing 10 (v/v)% PG" with Lactec Injection at a 2-fold common ratio.
3) LR containing 10 (v/v)% PG and 4.5 (w/v)% dextran was prepared by first dissolving 500 mg of Dextran 40 (Mw = 40000, manufactured by Tokyo Chemical Industry Co., Ltd.) in 10 mL of Lactec Injection to prepare LR containing 5 (w/v)% dextran, and then mixing the LR containing 5 (w/v)% dextran and the above PG at a ratio of 9:1.
4) LR containing 5 (v/v)% PG and 4.75 (w/v)% dextran; LR containing 2.5 (v/v)% PG and 4.88 (w/v)% dextran; and LR containing 1.25 (v/v)% PG and 4.94 (w/v)% dextran were prepared by diluting the above "LR containing 10 (v/v)% PG and 4.5 (w/v)% dextran" with the above "LR containing 5 (w/v)% dextran" at a 2-fold common ratio.
5) LR containing 10 (v/v)% PG and 2.7 (w/v)% trehalose was prepared by mixing 0.5 mL of the above PG and 0.5 mL of Cellstor (registered trademark)-W (manufactured by Otsuka Pharmaceutical Factory, Inc.) (that is, lactated Ringer's solution containing 3 (w/v)% trehalose).
6) LR containing 5 (v/v)% PG and 2.85 (w/v)% trehalose; LR containing 2.5 (v/v)% PG and 2.925 (w/v)% trehalose; and LR containing 1.25 (v/v)% PG and 2.9625 (w/v)% trehalose were prepared by diluting the above "LR containing 10 (v/v)% PG and 2.7 (w/v)% trehalose" with Cellstor-W at a 2-fold common ratio.
7) LR containing 10 (v/v)% PG, 2.7 (w/v)% trehalose, and 4.5 (w/v)% dextran used was the one prepared in 3) of Study 1 described above.
8) LR containing 5 (v/v)% PG, 2.85 (w/v)% trehalose, and 4.75 (w/v)% dextran; LR containing 2.5 (v/v)% PG, 2.925 (w/v)% trehalose, and 4.875 (w/v)% dextran; and LR containing 1.25 (v/v)% PG, 2.9625 (w/v)% trehalose, and 4.9375 (w/v)% dextran were prepared by diluting the above "LR containing 10 (v/v)% PG, 2.7 (w/v)% trehalose, and 4.5 (w/v)% dextran" with Cellstor-S at a 2-fold common ratio.

### [Preparation of liquids for cryopreserving cell used in Study 4]

1) 4 base liquids comprising 5 (v/v)% PG (physiological saline [S], Ringer's solution [R], acetated Ringer's solution [AR], and lactated Ringer's solution [LR]) were prepared by mixing 0.5 mL of the above PG and 9.5 mL of 4 base liquids (OTSUKA NORMAL SALINE [manufactured by Otsuka Pharmaceutical Factory, Inc.], Ringer's Solution "OTSUKA" [manufactured by Otsuka Pharmaceutical Factory, Inc.], Veen-F Injection [manufactured by Fuso Pharmaceutical Industries, Ltd.], and Lactec Injection).
2) S containing 5 (v/v)% PG and 4.75 (w/v)% dextran was prepared by first dissolving 500 mg of Dextran 40 (Mw = 40000, manufactured by Tokyo Chemical Industry Co., Ltd.) in 10 mL of Lactec Injection to prepare LR containing 5 (w/v)% dextran, and then mixing 0.5 mL of the above PG and 9.5 mL of the above "LR containing 5 (w/v)% dextran."
3) R containing 5 (v/v)% PG and 4.75 (w/v)% dextran was prepared by first dissolving 500 mg of the above Dextran 40 in 10 mL of Ringer's Solution "OTSUKA" to prepare R containing 5 (w/v)% dextran, and then mixing 0.5 mL of the above PG and 9.5 mL of the R containing 5 (w/v)% dextran.
4) AR containing 5 (v/v)% PG and 4.75 (w/v)% dextran was prepared by first dissolving 500 mg of the above Dextran 40 in 10 mL of Veen-F Injection to prepare AR containing 5 (w/v)% dextran, and then mixing 0.5 mL of the above PG and 9.5 mL of the AR containing 5 (w/v)% dextran.
5) LR containing 5 (v/v)% PG and 4.75 (w/v)% dextran was prepared by first dissolving 500 mg of the above Dextran 40 in 10 mL of Lactec Injection to prepare LR containing 5 (w/v)% dextran, and then mixing 0.5 mL of the above PG and 9.5 mL of the LR containing 5 (w/v)% dextran.

### [Preparation of liquids for cryopreserving cell used in Study 5]

1) LR containing 10 (v/v)% PG and 4.5 (w/v)% dextran; LR containing 5 (v/v)% PG and 4.75 (w/v)% dextran; LR containing 2.5 (v/v)% PG and 4.88 (w/v)% dextran; and LR containing 1.25 (v/v)% PG and 4.94 (w/v)% dextran used were those prepared in 3) and 4) of the above [Preparation of liquids for cryopreserving cell used in Study 3].
2) LR containing 7.5 (v/v)% PG and 4.63 (w/v)% dextran was prepared by mixing the above "LR containing 5 (v/v)% PG and 4.75 (w/v)% dextran" and the above "LR containing 10 (v/v)% PG and 4.5 (w/v)% dextran" at a ratio of 1:1.
3) LR containing 3.75 (v/v)% PG and 4.81 (w/v)% dextran was prepared by mixing the above "LR containing 2.5 (v/v)% PG and 4.88 (w/v)% dextran" and the above "LR containing 5 (v/v)% PG and 4.75 (w/v)% dextran" at a ratio of 1:1.
4) LR containing 6.25 (v/v)% PG and 4.69 (w/v)% dextran was prepared by mixing the above "LR containing 5 (v/v)% PG and 4.75 (w/v)% dextran" and the above "LR containing 7.5 (v/v)% PG and 4.63 (w/v)% dextran" at a ratio of 1:1.
5) LR containing 8.75 (v/v)% PG and 4.56 (w/v)% dextran was prepared by mixing the above "LR containing 7.5 (v/v)% PG and 4.63 (w/v)% dextran" and the above "LR containing 10 (v/v)% PG and 4.5 (w/v)% dextran" at a ratio of 1:1.

### [Preparation of liquids for cryopreserving cell used in Study 6]

1) LR containing 4 (v/v)% PG was prepared by mixing the above "LR containing 10 (v/v)% PG" and Lactec Injection at a ratio of 4:6.
2) LR containing 4 (v/v)% PG and 9.6 (w/v)% dextran was prepared by first dissolving 1 g of the above Dextran 40 in 10 mL of Lactec Injection to prepare LR containing 10 (w/v)% dextran, and then mixing the LR containing 10 (w/v)% dextran and the above PG at a ratio of 96:4.
3) LR containing 4 (v/v)% PG and 4.8 (w/v)% dextran; LR containing 4 (v/v)% PG and 2.4 (w/v)% dextran; and LR containing 4 (v/v)% PG and 1.2 (w/v)% dextran were prepared by diluting the above "LR containing 4 (v/v)% PG and 9.6 (w/v)% dextran" with the above "LR containing 4 (v/v)% PG" at a 2-fold common ratio.
4) LR containing 4 (v/v)% PG and 7.2 (w/v)% dextran was prepared by mixing the above "LR containing 4 (v/v)% PG and 4.8 (w/v)% dextran" and the above "LR containing 4 (v/v)% PG and 9.6 (w/v)% dextran" at a ratio of 1:1.

### [Preparation of liquids for cryopreserving cell used in Study 7]

1) LR containing 10 (v/v)% PG used was the one prepared in 1) of the above [Preparation of liquids for cryopreserving cell used in Study 3].
2) LR containing 4 (v/v)% PG used was the one prepared in 1) of the above [Preparation of liquids for cryopreserving cell used in Study 6].
3) LR containing 10 (v/v)% PG and 4.5 (w/v)% dextran was prepared by mixing the above "LR containing 5 (w/v)% dextran" and the above PG at a ratio of 9:1.
4) LR containing 4 (v/v)% PG and 4.8 (w/v)% dextran was prepared by mixing the above "LR containing 10 (v/v)% PG and 4.5 (w/v)% dextran" and the above "LR containing 5 (w/v)% dextran" at a ratio of 4:6.
5) LR containing 10 (v/v)% PG and 4.5% hydroxyethyl starch (HES) was prepared by first dissolving 500 mg of HES (Mw = 2220339 [measured value]) (manufactured by Fresenius KabiAustria GmbH) in 10 mL of Lactec Injection to prepare LR containing 5 (w/v)% HES, and then mixing the LR containing 5 (w/v)% HES and the above PG at a ratio of 9:1.
6) LR containing 4 (v/v)% PG and 4.8 (w/v)% HES was prepared by mixing the above "LR containing 10 (v/v)% PG and 4.5 (w/v)% HES" and the above "LR containing 5 (w/v)% HES" at a ratio of 4:6.
7) LR containing 10 (v/v)% PG and 2.7 (w/v)% trehalose used was the one prepared in 5) of the above [Preparation of liquids for cryopreserving cell used in Study 3].
8) LR containing 4 (v/v)% PG and 2.88 (w/v)% trehalose was prepared by mixing the above "LR containing 10 (v/v)% PG and 2.7 (w/v)% trehalose" and Cellstor-W at a ratio of 4:6.
9) LR containing 10 (v/v)% PG, 2.7 (w/v)% trehalose, and 4.5 (w/v)% dextran was prepared by first dissolving 500 mg of the above Dextran 40 in 10 mL of Cellstor-W to prepare Cellstor-W containing 5 (w/v)% dextran, and then mixing the Cellstor-W containing 5 (w/v)% dextran and the above PG at a ratio of 9:1.
10) LR containing 4 (v/v)% PG, 2.88 (w/v)% trehalose, and 4.8 (w/v)% dextran was prepared by mixing the above "LR containing 10 (v/v)% PG, 2.7 (w/v)% trehalose, and 4.5 (w/v)% dextran" and the above "Cellstor-W containing 5 (w/v)% dextran" at a ratio of 4:6.
11) LR containing 10 (v/v)% PG, 2.7 (w/v)% trehalose, and 4.5 (w/v)% HES was prepared by first dissolving 500 mg of the above HES in 10 mL of Cellstor-W to prepare Cellstor-W containing 5 (w/v)% HES, and then mixing the Cellstor-W containing 5 (w/v)% HES and the above PG at a ratio of 9:1.
12) LR containing 4 (v/v)% PG, 2.88 (w/v)% trehalose, and 4.8 (w/v)% HES was prepared by mixing the above "LR containing 10 (v/v)% PG, 2.7 (w/v)% trehalose, and 4.5 (w/v)% HES" and the above "Cellstor-W containing 5 (w/v)% HES" at a ratio of 4:6.

### [Preparation of liquids for cryopreserving cell used in Study 8]

1) Lactated Ringer's solution (LR) containing 2.5 (v/v)% PG was prepared by mixing Lactec Injection and PG at a ratio of 39:1.
2) LR containing 2.5 (v/v)% PG and 2.925 (w/v)% trehalose was prepared by mixing Cellstor-W and the above PG at a ratio of 39:1.
3) LR containing 2.5 (v/v)% PG, 2.925 (w/v)% trehalose, and 4.875 (w/v)% dextran was prepared by mixing Cellstor-S and the above PG at a ratio of 39:1.

### [Preparation of liquids for cryopreserving cell used in Study 9]

1) LR containing 4 (v/v) % PG used was the one prepared in 1) of the above [Preparation of liquids for cryopreserving cell used in Study 6].
2) LR containing 4 (v/v)% PG and 1.44 (w/v)% glucose was prepared by first dissolving 150 mg of D(+)-glucose (manufactured by FUJIFILM Wako Pure Chemical Corporation) in 10 mL of Lactec Injection to prepare LR containing 1.5 (w/v)% glucose, and then mixing 0.4 mL of the above PG and 9.6 mL of the LR containing 1.5 (w/v)% glucose.
3) LR containing 4 (v/v)% PG and 1.44 (w/v)% fructose was prepared by first dissolving 150 mg of D(-)-fructose (manufactured by FUJIFILM Wako Pure Chemical Corporation) in 10 mL of Lactec Injection to prepare LR containing 1.5 (w/v)% fructose, and then mixing 0.4 mL of the above PG and 9.6 mL of the LR containing 1.5 (w/v)% fructose.
4) LR containing 4 (v/v)% PG and 2.88 (w/v)% trehalose was prepared by first dissolving 332 mg of trehalose dihydrate (manufactured by FUJIFILM Wako Pure Chemical Corporation) in 10 mL of Lactec Injection to prepare LR containing 3 (w/v)% trehalose, and then mixing 0.4 mL of the above PG and 9.6 mL of the LR containing 3 (w/v)% trehalose.
5) LR containing 4 (v/v)% PG and 2.88% sucrose was prepared by first dissolving 300 mg of sucrose (manufactured by FUJIFILM Wako Pure Chemical Corporation) in 10 mL of Lactec Injection to prepare LR containing 3% sucrose, and then mixing 0.4 mL of the above PG and 9.6 mL of the LR containing 3% sucrose.
6) LR containing 4 (v/v)% PG and 2.88 (w/v)% lactose was prepared by first dissolving 316 mg of lactose monohydrate (manufactured by FUJIFILM Wako Pure Chemical Corporation) in 10 mL of Lactec Injection to prepare LR containing 3 (w/v)% lactose, and then mixing 0.4 mL of the above PG and 9.6 mL of the LR containing 3 (w/v)% lactose.

### [Preparation of liquids for cryopreserving cell used in Study 10]

1) LR containing 4 (v/v) % PG used was the one prepared in 1) of the above [Preparation of liquids for cryopreserving cell used in Study 6].
2) LR containing 4 (v/v)% PG and 11.52 (w/v)% trehalose was prepared by first dissolving 1.328 g of the above trehalose dihydrate in 10 mL of Lactec Injection to prepare LR containing 12 (w/v)% trehalose, and then mixing the LR containing 12 (w/v)% trehalose and the above PG at a ratio of 96:4.
3) LR containing 4 (v/v)% PG and 5.76 (w/v)% trehalose; LR containing 4 (v/v)% PG and 2.88 (w/v)% trehalose; LR containing 4 (v/v)% PG and 1.44 (w/v)% trehalose; and LR containing 4 (v/v) % PG and 0.72 (w/v)% trehalose were prepared by diluting the above "LR containing 4 (v/v)% PG and 11.52 (w/v)% trehalose" with the above "LR containing 4 (v/v)% PG" at a 2-fold common ratio.

### [Preparation of liquids for cryopreserving cell used in Study 11]

3 base liquids (culture medium containing serum, LR containing trehalose, and LR) comprising 4 (v/v)% PG were prepared by mixing MEMα (manufactured by Gibco) containing 10% FBS (manufactured by Gibco), Cellstor-W, and Lactec Injection, respectively, with the above PG at a ratio of 24:1.

### [Preparation of liquids for cryopreserving cell used in Studies 12 to 15]

LR containing 2 (v/v)% PG, 2.7 (w/v)% trehalose, and 4.5 (w/v)% dextran; LR containing 3 (v/v)% PG, 2.7 (w/v)% trehalose, and 4.5 (w/v)% dextran; LR containing 4 (v/v)% PG, 2.7 (w/v)% trehalose, and 4.5% (w/v)% dextran; LR containing 5 (v/v)% PG, 2.7 (w/v)% trehalose, and 4.5 (w/v)% dextran; and LR containing 7.5 (v/v)% PG, 2.7 (w/v)% trehalose, and 4.5 (w/v)% dextran were prepared by first mixing Cellstor-S and the above PG at a ratio of 9:1 to prepare LR containing 10 (v/v)% PG, 2.7 (w/v)% trehalose, and 4.5 (w/v)% dextran, and then mixing Cellstor-S and the "LR containing 10 (v/v)% PG, 2.7 (w/v)% trehalose, and 4.5 (w/v)% dextran" at ratios of 4:1, 7:3, 3:2, and 1:1, respectively.

### [Culture of hAD-MSCs (Studies 1 to 10)]

Human adipose-derived mesenchymal stem cells (hAD-MSCs) (manufactured by Lonza Walkersville) were cultured according to the human adipocyte-derived stem cell culture protocol of Lonza Walkersville. Specifically, hAD-MSCs were seeded in 75 cm² flasks provided with 15 mL of a medium prepared from a medium kit (PT-4505, ADSC BulletKit, manufactured by Lonza Walkersville) (hereinafter referred to as the "MSC medium"), and cultured at 37°C in a 5% CO₂ incubator. Medium exchange was carried out every 3 days or 4 days. The cells were passaged to a confluence of about 90% (a confluence of 80 to 100%), and the flasks after 2 to 5 passages were used in the method described in the following section "Preparation of hAD-MSCs (Studies 1 to 10) . "

### [Preparation of hAD-MSCs (Studies 1 to 10)]

hAD-MSCs were prepared according to the following procedures [1] to [11].
[1] A personal incubator (PIC-100, manufactured by As One Corporation) was set at 37°C and heated.
[2] The 75 cm² flasks in which hAD-MSCs were cultured were removed from the CO₂ incubator.
[3] The state of the cells was observed under a microscope, and cells that were 80% to 100% confluent were used.
[4] The MSC medium was aspirated, and 8 mL of PBS(-) (product number: 14190-144, manufactured by GIBCO) was added to each 75 cm² flask.
[5] PBS(-) was aspirated, and then 4 mL of trypsin/EDTA (product code: CC-5012, manufactured by Lonza Walkersville) was added to each flask and incubated for 5 minutes under a condition of 37 ± 2°C in the personal incubator.
[6] The flasks were gently shaken while observing the cells under a microscope until about 90% of the cells were detached.
[7] The trypsin reaction was stopped by adding 8 mL of TNS (trypsin neutralizing solution, product code: CC-5002, manufactured by Lonza Walkersville), and cells were detached by pipetting and transferred into 50 mL ProteoSave (MS-52550, Sumitomo Bakelite Co., Ltd.).
[8] The cells were recovered by centrifugation at 210 × g for 5 minutes at room temperature.
[9] The supernatant was aspirated, and the cells were suspended in CellStor-W.
[10] 20 µL of the resulting cell suspension was taken, mixed with 20 µL of 0.4% Trypan Blue (item number: 15250-061, manufactured by gibco), and the number of cells (living cells and dead cells) was measured by using a cell counting chamber (One Cell Counter, item number: OCC01, manufactured by Bio Medical Science Inc.). The number of cells measured was the number of cells in the four corner areas of one area measured by using One Cell Counter.
[11] The total cell concentration was calculated by using the following expression. Expression: Total cell concentration (cells/mL) = (total number of cells measured × 10⁴)/4 × 2

### [Culture of hCD8-positive T cells (Studies 11 and 12)]

1.5 × 10⁶ human CD8-positive T cells (hCD8-positive T cells) (manufactured by STEMCELL Technologies) were seeded in a flask containing a culture medium for T lymphocyte culture (product name: TLY CULTURE Kit 25, TLY-FK25, manufactured by GC Lymphotec Inc.), and culture was started at 37°C in a 5% CO₂ incubator. On day 3 after culture, the cells were transferred to 8 or 10 25 cm² flasks. After that, the flasks on day 9 after culture were used in the method described in the following section [Preparation of hCD8-positive T cells (Studies 11 and 12)].

### [Preparation of hCD8-positive T cells (Studies 11 and 12)]

hCD8-positive T cells were prepared according to the following procedures [1] to [4].
[1] The hCD8-positive T cells were transferred from the flasks to 50 mL ProteoSave (MS-52550, manufactured by Sumitomo Bakelite Co., Ltd.).
[2] The cells were recovered by centrifugation at 300 × g for 10 minutes at room temperature.
[3] The supernatant was aspirated, and the cells were suspended in Lactec Injection.
[4] 100 µL of the resulting cell suspension was aliquoted and sampled by using a Via2-Cassette (manufactured by Chemometec), and the total number of cells was measured by using NucleoCounter (manufactured by Chemometec) .

### [Culture of hCD8-positive T cells and hCD4-positive T cells (Studies 13 and 14)]

1.5 × 10⁶ human CD8-positive T cells (hCD8-positive T cells) (manufactured by STEMCELL Technologies) or human CD4-positive T cells (hCD4-positive T cells) (manufactured by Lonza Walkersville) were seeded in a flask containing a culture medium for T lymphocyte culture (product name: TLY CULTURE Kit 25, TLY-FK25, manufactured by GC Lymphotec Inc.), and culture was started at 37°C in a 5% CO₂ incubator. On day 3 after culture, the hCD8-positive T cells or the hCD4-positive T cells were each transferred to three 75 cm² flasks. On day 7 and day 9 after culture, one flask each was used in the method described in the following section [Preparation of hCD8-positive T cells and hCD4-positive T cells (Studies 13 and 14)]. Furthermore, on day 10 after culture, cells were recovered from the one remaining flask and divided into three 75 cm² flasks, and 10 mL of a T cell medium (product name: X-VIVO15 serum-free lymphocyte medium, 04-418Q, manufactured by Lonza Walkersville) was added to each flask. After that, the three flasks on day 11 after culture were used in the method described in the following section [Preparation of hCD8-positive T cells and hCD4-positive T cells (Studies 13 and 14)].

### [Preparation of hCD8-positive T cells and hCD4-positive T cells (Studies 13 and 14)]

hCD8-positive T cells were prepared according to the following procedures [1] to [5].
[1] The hCD8-positive T cells or the hCD4-positive T cells were transferred from the flasks to 50 mL ProteoSave (MS-52550, manufactured by Sumitomo Bakelite Co., Ltd.).
[2] The cells were recovered by centrifugation at 300 × g for 10 minutes at room temperature.
[3] The supernatant was aspirated, and the cells were suspended in CellStor-W.
[4] 20 µL of the resulting cell suspension was taken, mixed with 20 µL of 0.4% Trypan Blue (item number: 15250-061, manufactured by gibco), and the number of cells (living cells and dead cells) was measured by using a cell counting chamber (One Cell Counter, item number: OCC01, manufactured by Bio Medical Science Inc.). The number of cells measured was the number of cells in the four corner areas of one area measured by using One Cell Counter. [5] The total cell concentration was calculated by using the following expression. Expression: Total cell concentration (cells/mL) = (total number of cells measured × 10⁴)/4 × 2

### [Culture of hCD34-positive hematopoietic stem cells (Study 15)]

Hematopoietic Progenitor Cell Expansion Medium XF (manufactured by Takara Bio Inc.) comprising 2 × 10⁴ cells/mL of umbilical cord blood-derived human CD34-positive hematopoietic stem cells (hCD34-positive hematopoietic stem cells) (manufactured by Takara Bio Inc.) was seeded in a 75 cm² flask for suspension culture (product name: Super Quality Suspension Cell Culture Flask 75 cm² Filter Cap, MS-2325RS, manufactured by Sumitomo Bakelite Co., Ltd.), and culture was started at 37°C in a 5% CO₂ incubator. On day 2 or 3 after culture, an equal amount of Hematopoietic Progenitor Cell Expansion Medium XF was added, and culture was carried out for an additional 3 days. 1) The Medium was transferred to 50 mL ProteoSave (MS-52550, manufactured by Sumitomo Bakelite Co., Ltd.) and centrifuged at 240 × g for 10 minutes at room temperature to recover cells. 2) The supernatant was aspirated, fresh Hematopoietic Progenitor Cell Expansion Medium XF was added, the cells were suspended, and then the resulting suspension was seeded in a fresh 75 cm² flask for suspension culture. The above operations 1) and 2) were repeated every 2 or 3 days, and the flask on day 12 was used in the method described in the following section [Preparation of hCD34-positive hematopoietic stem cells (Study 15)].

### [Preparation of hCD34-positive hematopoietic stem cells (Study 15)]

hCD34-positive hematopoietic stem cells were prepared according to the following procedures [1] to [4].
[1] The hCD34-positive hematopoietic stem cells were transferred from the flask to 50 mL ProteoSave (MS-52550, manufactured by Sumitomo Bakelite Co., Ltd.).
[2] The cells were recovered by centrifugation at 240 × g for 10 minutes at room temperature.
[3] The supernatant was aspirated, and the cells were suspended in Lactec Injection.
[4] 100 µL of the resulting cell suspension was taken and sampled by using a Via2-Cassette (manufactured by Chemometec), and the total number of cells was measured by using NucleoCounter (manufactured by Chemometec).

### [Cryopreservation of mammalian cells]

4 mammalian cells (hAD-MSCs, hCD8-positive T cells, hCD4-positive T cells, and hCD34-positive hematopoietic stem cells) were cryopreserved according to the following procedures [1] to [4].
[1] The various mammalian cells suspended in Cellstor-W or Lactec Injection were transferred to 15 mL STEMFULL (article number: MS-90150, size: 15 mL, Sumitomo Bakelite Co., Ltd.).
[2] A centrifugation treatment (for hAD-MSCs, 210 × g, 5 minutes, room temperature; for hCD8-positive T cells and hCD4-positive T cells, 300 × g, 10 minutes, room temperature; for hCD34-positive hematopoietic stem cells, 240 × g, 10 minutes, room temperature) was carried out, and the supernatant was aspirated.
[3] The various mammalian cells were resuspended in various liquids for cryopreserving a cell in such a way as to provide 1.0 × 10⁶ cells/mL, and 1 mL each of the resulting suspensions was placed in vials (Nunc CryoTube Vials, article number: 375418, size: 1.8 mL, manufactured by Thermo Fisher Scientific), respectively.
[4] The vials were placed in BICELL (BIO FREEZING VESSEL) (manufactured by Nihon Freezer Co., Ltd.) and preserved in a -80°C freezer until the next day, thereafter the vials were transferred from BICELL to a sample box, and the sample box was transferred into a liquid nitrogen tank (Thermolyne Locator Plus Liquid Nitrogen Cryopreservation Vessel Type 509, CS509X21A-70, manufactured by Toei Kaisha, Ltd.) and preserved for any period of time.

### [Preparation of samples of cells after freezing and thawing]

Samples for evaluating the viability of the 4 mammalian cells (hAD-MSCs, hCD8-positive T cells, hCD4-positive T cells, and hCD34-positive hematopoietic stem cells) after freezing and thawing were prepared according to the following procedures [1] to [6].
[1] A constant temperature bath (water bath) was set at 37°C and heated.
[2] The frozen vials containing various liquids for cryopreserving a cell comprising various mammalian cells, respectively, were taken out of the liquid nitrogen tank and transferred to a container containing dry ice.
[3] The vials were quickly transferred to the constant temperature bath set at 37°C, and were thawed with gentle stirring until only a few pieces of ice remained.
[4] Water was quickly removed from the surface of the vials, and then the vials were wiped with 70% ethanol and transferred to a safety cabinet.
[5] After thawing, the cells were suspended (gently pipetting 5 times at a liquid volume of 500 µL by using a 1 mL pipette) with the end of the tip thereof inserted to a position visually about 5 mm from the bottom.
[6] 50 µL and 20 µL of the resulting suspension were aliquoted as a cell suspension for annexin V positivity rate evaluation and a cell suspension for trypan blue staining, respectively, 100 µL thereof was aliquoted as a cell suspension for NucleoCounter analysis, and 800 µL of the hCD34-positive hematopoietic stem cell suspension was aliquoted for CD34 positivity rate evaluation. In order to carry out Studies 11 and 12, the aliquoted cell suspensions were subjected to shaking preservation (100 rpm, 25 mm) at room temperature.

### [Annexin V positivity rate of mammalian cells after freezing and thawing]

The annexin V positivity rate of the 4 mammalian cells (hAD-MSCs, hCD8-positive T cells, hCD4-positive T cells, and hCD34-positive hematopoietic stem cells) after freezing and thawing was evaluated according to the following procedures [1] to [6].
[1] Annexin Binding Buffer (×10) included in an apoptosis detection kit (Annexin V-FITC Kit, manufactured by nacalai tesque) was diluted 10-fold with OTSUKA DISTILLED WATER to prepare a liquid for annexin V staining, which was then ice cooled.
[2] 100 µL of a Ca chloride correction liquid (1 mEq/mL) was diluted 10-fold with 900 µL of OTSUKA DISTILLED WATER to prepare a Ca chloride diluted liquid.
[3] The 50 µL aliquot of the cell suspension for annexin V positivity rate evaluation was transferred to a round tube.
[4] 3 µL of the Ca chloride diluted liquid was added to each cell suspension such that the calcium ion concentration was 1.5 mM or more.
[5] 1 µL of Annexin V-FITC was added and mixed, and staining was carried out on ice in the dark for 15 minutes.
[6] After staining, 400 µL of the liquid for annexin V staining was added, and the resulting mixture was transferred to a round-bottom standard tube through a cell strainer, and the annexin V positivity rate was rapidly evaluated by using flow cytometry (Gallios, manufactured by Beckman Coulter). The annexin V positivity rate was analyzed and calculated by using Kaluza Analysis Software (Ver. 1.5a, manufactured by Beckman Coulter).

### [Trypan blue staining (Studies 2, 4 to 10, 13, and 14)]

The 20 µL aliquot of the cell suspension for trypan blue staining was mixed with 20 µL of a trypan blue staining liquid, and optical microscopes (ECLIPSE TS100, manufactured by Nikon Corporation, and OLYMPUS CKX53, manufactured by Olympus Corporation) were used to measure each of the total number of cells and the number of dead cells with a One Cell Counter. Then, these measured values were input into the following expressions (cell viability (%) = [total number of cells - number of dead cells]/total number of cells × 100) and (viable cell recovery rate (%) = number of viable cells in 1 mL at each point after freezing/number of viable cells in 1 mL before freezing × 100) to calculate the cell viability and the viable cell recovery rate.

### [NucleoCounter analysis (Studies 11, 12, and 15)]

The 100 µL aliquot of the cell suspension for NucleoCounter analysis was sampled by using a Via2-Cassette (manufactured by Chemometec), and the total number of cells and the number of dead cells were each measured by using NucleoCounter (manufactured by Chemometec). Then, these measured values were input into the following expressions (cell viability (%) = [total number of cells - number of dead cells]/total number of cells × 100) and (viable cell recovery rate (%) = number of viable cells in 1 mL at each point after freezing/number of viable cells in 1 mL before freezing × 100) to calculate the cell viability and the viable cell recovery rate.

### [Cell proliferation ability of hAD-MSCs after freezing and thawing]

hAD-MSCs immediately after freezing and thawing were seeded in a 6-well plate at a density of 5.6 × 10³ cells/cm² (5.0 × 10⁴ cells/9 cm²/well) and cultured by using the MSC medium. Medium exchange was carried out within 24 hours after seeding and on day 3 or 4 thereafter. On day 1, day 3, day 5, and day 7 after seeding, the 6-well plate in which the hAD-MSCs were cultured was washed with PBS(-) and enzymatically treated with trypsin/EDTA, the trypsin reaction was stopped with TNS, the number of cells after re-culture was measured with One Cell Counter, and the cell proliferation ability of the hAD-MSCs after freezing and thawing was evaluated.

### [Evaluation of CD34 positivity rate of hCD34-positive hematopoietic stem cells]

The CD34 positivity rate of hCD34-positive hematopoietic stem cells after freezing and thawing was evaluated according to the following procedures [1] to [9].
[1] 800 µL of the hCD34-positive hematopoietic stem cell suspension was divided into 500 µL (sample for staining) and 300 µL (sample for control) aliquots, which were then each transferred to 1.5 mL ProteoSave (MS4265M, manufactured by Sumitomo Bakelite Co., Ltd.).
[2] A centrifugation treatment (240 × g, 10 minutes, room temperature) was carried out, and the supernatant was aspirated.
[3] 49 µL and 30 µL of Stain Buffer (FBS) were added to the sample for staining and the sample for the control, respectively, to suspend the cells.
[4] 1 µL of PE-CD34 Antibody (manufactured by Milteny) was added to the sample for staining and mixed.
[5] The resulting mixture was incubated (antigen-antibody reaction) for 10 minutes in a refrigerator, with exclusion of light.
[6] 1 mL of Stain Buffer (FBS) was added to each of the sample for staining and the sample for the control, which were then subjected to a centrifugation treatment (240 × g, 10 minutes, 4°C).
[7] The supernatant was aspirated, and then 1 mL of Stain Buffer (FBS) was added to each of the sample for staining and the sample for control, which was then subjected to a centrifugation treatment (240 × g, 10 minutes, 4°C).
[8] The supernatant was aspirated, then 500 µL of Stain Buffer (FBS) was added to suspend the cells, and the resulting suspension was transferred to a round-bottom standard tube through a cell strainer.
[9] Cells having a positive PE-derived fluorescence signal (that is, hCD34-positive hematopoietic stem cells) were detected by using a flow cytometer (Gallios, manufactured by Beckman Coulter), the proportion of CD34-positive cells to the total number of cells (CD34 positivity rate) was calculated by using the analysis software Kaluza (Ver. 1.5a, manufactured by Beckman Coulter), and then the relative value (value on the vertical axis in Figure 19B) when the CD34 positivity rate in the cells before cryopreservation was set to 1 was calculated.

### [Calculation method and statistical processing]

The mean value ± standard deviation of each group was determined. Statistical analysis was carried out by using SAS 9.4 (EXSUS Version 10.0). Student's t test, Dunnett's test, and Tukey's test were carried out if necessary, and if a value was less than a significance level of 5%, it was determined that there was a significant difference.

### 2. Results

### 2-1 Study 1

In order to study whether there was a difference in the proliferation ability of mammalian cells after freezing and thawing between when the mammalian cells were cryopreserved in a liquid for cryopreserving a cell comprising DMSO and when the mammalian cells were cryopreserved in a liquid for cryopreserving a cell comprising PG, hAD-MSCs were cryopreserved in each of a base liquid comprising 1.25 to 10% DMSO (LR containing 2.7% trehalose and 4.5% dextran) and in the above base liquid comprising 1.25 to 10% PG, the hAD-MSCs after freezing and thawing were cultured for 1 to 7 days, and changes in the number of cells over time were analyzed.

As a result, from day 5 of culture onward, the hAD-MSCs cryopreserved in the liquid for cryopreserving a cell comprising 1.25 to 10% DMSO showed no difference in cell proliferation efficiency due to differences in DMSO concentration (Figure 1A and Table 1). On the other hand, it was shown that the hAD-MSCs cryopreserved in the liquid for cryopreserving a cell comprising 2.5 to 5% PG proliferated significantly more efficiently than the hAD-MSCs cryopreserved in the liquid for cryopreserving a cell comprising 10% PG (Figure 1B and Table 1). In addition, it was shown that the hAD-MSCs cryopreserved in the liquid for cryopreserving a cell comprising 2.5 to 5% PG proliferated significantly more efficiently than the hAD-MSCs cryopreserved in the liquid for cryopreserving a cell comprising 2.5 to 5% DMSO (Figures 2B and 2C).

These results show that when mammalian cells are cryopreserved in a liquid for cryopreserving a cell containing 2.5 to 5% PG, trehalose, and dextran, the proliferation ability of the mammalian cells after freezing and thawing can be more effectively increased than when mammalian cells are cryopreserved in a liquid for cryopreserving a cell containing PG having a concentration of other than 2.5 to 5%, or trehalose, and dextran and when mammalian cells are cryopreserved in a liquid for cryopreserving a cell containing DMSO, trehalose, and dextran.

**[Table 1]**

| Liquid for cryopreserving a cell | Number of cells (×10⁴ cells/well) | | | | |
|---|---|---|---|---|---|
| | Immediately after freezing and thawing | After freezing and thawing | | | |
| | | Day 1 | Day 3 | Day 5 | Day 7 |
| Base liquid (LR containing trehalose a nd dextran) | | | | | |
| 1.25%DMSO | 5.2±0.0 | 4.2±0.9 | 12.8±1.4 | 25.6±2.0 | 25.3±0.5 |
| 2.5%DMSO | 5.1±0.2 | 6.0±0.4 | 17.8±2.6 | 22.6±1.7 | 24.1±1.5 |
| 5%DMSO | 5.1±0.2 | 6.0±0.1 | 16.7±0.4 | 23.5±0.9 | 22.4±2.3 |
| 10%DMSO | 5.1±0.3 | 6.3±0.8 | 16.4±0.8 | 22.4±3.2 | 23.8±1.0 |
| 1.25%PG | 5.1±0.1 | 2.3±0.5 | 11.0±2.8 | 23.5±4.6 | 26.2±3.7 |
| 2.5%PG | 5.2±0.0 | 5.1±0.4 | 16.5±1.2 | 39.9±3.4 | 42.8±3.1 |
| 5%PG | 5.0±0.1 | 5.6±1.0 | 18.4±0.9 | 41.9±5.7 | 43.5±4.8 |
| 10%PG | 5.0±0.2 | 5.1±1.3 | 15.9±3.1 | 24.8±1.6 | 27.0±3.1 |

Figure 1 was created based on the results in Table 1.

### 2-2 Study 2

In order to compare and study the effect of using LR containing trehalose and dextran as a base liquid of a liquid for cryopreserving a cell comprising 2.5 to 5% PG with a liquid for cryopreserving a cell comprising DMSO, hAD-MSCs were cryopreserved in liquids for cryopreserving a cell containing trehalose and dextran and comprising 4% PG or 4% DMSO (LR containing 4% PG, 2.88% trehalose, and 4.8% dextran; and LR containing 4% DMSO, 2.88% trehalose, and 4.8% dextran), and the cell viability and the viable cell recovery rate of the hAD-MSCs immediately after freezing and thawing, as well as the cell proliferation ability thereof after freezing and thawing, were analyzed. As a comparative control, the cell proliferation ability of hAD-MSCs that were not cryopreserved was also analyzed in the same manner.

As a result, the cell viability and the viable cell recovery rate of the hAD-MSCs immediately after freezing and thawing were high when the hAD-MSCs were cryopreserved in any of the liquids for cryopreserving a cell (Table 2).

**[Table 2]**

| Liquid for cryopreserving a cell | Before cryopreservation | Immediately after freezing and thawing |
|---|---|---|
| Base liquid (LR contain ing trehalose and dextran) | | |
| | Cell viability (%) | |
| 4%PG | 98.8±0.7 | 94.7±1.7 |
| 4%DMSO | | 96.1±1.8 |

| | Viable cell recovery rate (%) | |
|---|---|---|
| 4%PG | 100.0 | 94.5±5.5 |
| 4%DMSO | | 95.5±6.4 |

In addition, as a result of culturing the hAD-MSCs after freezing and thawing for 1 to 7 days and analyzing changes in the number of cells over time, it was shown that the hAD-MSCs cryopreserved in the liquid for cryopreserving a cell containing trehalose and dextran and comprising 4% PG (LR containing 4% PG, 2.88% trehalose, and 4.8% dextran) proliferated at the same or a higher level of efficiency than the hAD-MSCs that were not cryopreserved (Figure 3 and Table 3). On the other hand, it was shown that the proliferation level of the hAD-MSCs cryopreserved in the liquid for cryopreserving a cell containing trehalose and dextran and comprising 4% DMSO (LR containing 4% DMSO, 2.88% trehalose, and 4.8% dextran) were significantly lower than that of the hAD-MSCs that were not cryopreserved, from day 3 of culture onward (Figure 3 and Table 3).

These results show that both the liquid for cryopreserving a cell comprising 4% PG and the liquid for cryopreserving a cell comprising 4% DMSO exert the suppressing effect on cell death caused by freezing and thawing of mammalian cells at the same level, whereas the effect that increases the proliferation ability of mammalian cells after freezing and thawing is higher in the liquid for cryopreserving a cell comprising 4% PG.

**[Table 3]**

| | Number of cells (×10⁴ cell s/well) | | | | |
|---|---|---|---|---|---|
| | Day 0 | Day 1 | Day 3 | Day 5 | Day 7 |
| - | 5.0±0.3 | 6.7±1.0 | 22.9±1.6 | 37.0±2.9 | 45.6±2.4 |
| Liquid for cryopreserving a cell | (Immediately after freezing and thawing) | | | | |

| Base liquid (LR containing trehalose and dextran) | | | | | |
|---|---|---|---|---|---|
| 4%PG | 5.0±0.4 | 5.5±0.9 | 20.8±2.7 | 43.5±6.9 | 47.6±5.7 |
| 4%DMSO | 4.9±0.4 | 6.1±1.0 | 19.1±2.3 | 28.8±2.7 | 28.3±2.3 |

Figure 3 was created based on the results in Table 3.

### 2-3 Study 3

In order to study whether there was a difference in the proliferation ability of mammalian cells after freezing and thawing between when the mammalian cells were cryopreserved in a liquid for cryopreserving a cell comprising 2.5 to 5% PG and when the mammalian cells were cryopreserved in a liquid for cryopreserving a cell comprising PG having a concentration of other than 2.5 to 5%, hAD-MSCs were cryopreserved in 4 base liquids comprising 1.25 to 10% PG (LR; LR containing dextran; LR containing trehalose; and LR containing trehalose and dextran), the hAD-MSCs after freezing and thawing were cultured for 1 to 7 days, and changes in the number of cells over time were analyzed.

As a result, as shown by the results of Study 1, it was shown that when any of the base liquids was used, the hAD-MSCs cryopreserved in the liquid for cryopreserving a cell comprising 2.5 to 5% PG proliferated more efficiently than the hAD-MSCs cryopreserved in the liquid for cryopreserving a cell comprising 10% PG (Figure 4 and Table 4). In particular, when 3 base liquids (LR containing dextran; LR containing trehalose; and LR containing trehalose and dextran) were used, the cell proliferation ability of the hAD-MSCs was significantly higher (Figures 4B to 4D and Table 4).

These results show that when mammalian cells are cryopreserved in a liquid for cryopreserving a cell containing 2.5 to 5% PG, the proliferation ability of the mammalian cells after freezing and thawing can be more effectively increased than when mammalian cells are cryopreserved in a liquid for cryopreserving a cell containing PG having a concentration of other than 2.5 to 5%.

**[Table 4]**

| Liquid for cryopreserving a cell | Number of cells (×10⁴ cells/well) | | | | |
|---|---|---|---|---|---|
| | Immediately after freezing and thawing | After freezing and thawing | | | |
| | | Day 1 | Day 3 | Day 5 | Day 7 |
| Base liquid (LR) | | | | | |
| 1.25%PG | 5.0±0.5 | 0.9±0.7 | 4.5±3.3 | 18.8±13.0 | 20.4±9.2 |
| 2.5%PG | 5.2±0.4 | 3.0±0.8 | 11.9±2.2 | 33.3±7.3 | 38.9±2.4 |
| 5%PG | 5.3±0.3 | 3.3±0.7 | 16.1±0.7 | 35.2±3.0 | 39.6±4.1 |
| 10%PG | 5.5±0.3 | 3.4±0.5 | 15.2±1.6 | 29.9±1.2 | 30.7±2.3 |

| Base liquid (LR containing dextran) | | | | | |
|---|---|---|---|---|---|
| 1.25%PG | 5.3±0.3 | 1.8±0.5 | 9.5±5.3 | 27.3±8.1 | 33.2±3.3 |
| 2.5%PG | 5.5±0.2 | 3.8±0.5 | 16.5±0.3 | 45.5±3.5 | 46.8±6.3 |
| 5%PG | 5.1±0.3 | 3.9±0.3 | 19.2±2.9 | 41.6±2.2 | 46.4±3.2 |
| 10%PG | 5.3±0.3 | 4.1±0.5 | 18.2±3.2 | 33.8±1.7 | 32.3±0.4 |
| Base liquid (LR containing trehalose) | | | | | |
| 1.25%PG | 4.8±0.3 | 0.9±0.1 | 5.1±1.0 | 18.9±4.7 | 24.7±1.7 |
| 2.5%PG | 5.1±0.2 | 2.3±0.8 | 7.9±1.4 | 27.3±4.6 | 31.3±2.3 |
| 5%PG | 5.1±0.1 | 3.6±0.6 | 12.8±2.0 | 33.1±4.0 | 35.3±3.4 |
| 10%PG | 5.0±0.1 | 3.6±0.5 | 14.4±0.6 | 22.2±1.1 | 24.1±1.7 |

| Base liquid (LR containing trehalose and dextran) | | | | | |
|---|---|---|---|---|---|
| 1.25%PG | 5.2±0.2 | 2.4±1.1 | 11.2±1.2 | 28.3±3.3 | 30.6±2.8 |
| 2.5%PG | 5.3±0.0 | 4.7±0.6 | 15.7±0.7 | 39.3±3.5 | 40.7±4.2 |
| 5%PG | 5.1±0.1 | 5.4±0.5 | 19.0±0.5 | 38.0±5.4 | 42.6±4.0 |
| 10%PG | 4.9±0.2 | 3.9±0.4 | 13.6±1.7 | 24.6±2.0 | 26.9±0.7 |

Figure 4 was created based on the results in Table 4.

### 2-4 Study 4

In order to study a base liquid in a liquid for cryopreserving a cell comprising PG, hAD-MSCs were cryopreserved in 8 liquids for cryopreserving a cell (4 base liquids comprising 5% PG [physiological saline, Ringer's solution, acetated Ringer's solution, and lactated Ringer's solution] and the above 4 base liquids comprising 5% PG and 4.75% dextran), and the cell viability, the viable cell recovery rate, the annexin V positivity rate, and the cell proliferation ability of the hAD-MSCs immediately after thawing were analyzed.

As a result, in all measurement results of the cell viability, the viable cell recovery rate, and the annexin V positivity rate of the hAD-MSCs, when the liquids for cryopreserving a cell comprising 5% PG and the liquids for cryopreserving a cell comprising 5% PG and 4.75% dextran were used, no significant difference was observed among the above 4 base liquids in each (Figure 5 and Table 5). In addition, the cell viability of the hAD-MSCs when using the liquids for cryopreserving a cell comprising 5% PG and 4.75% dextran was higher than the cell viability of the hAD-MSCs when using the liquids for cryopreserving a cell comprising 5% PG together with the above 4 base liquids, and in particular, when Ringer's solution or acetated Ringer's solution was used as the base liquid, the cell viability was significantly higher (Figure 5A and Table 5). The viable cell recovery rate of the hAD-MSCs had a similar tendency, and in particular, when acetated Ringer's solution was used as the base liquid, the viable cell recovery rate was significantly higher (Figure 5B and Table 5). On the other hand, the annexin V positivity rate of the hAD-MSCs when using the liquids for cryopreserving a cell comprising 5% PG and 4.75% dextran was lower than the cell viability when using the liquids for cryopreserving a cell comprising 5% PG together with the above 4 base liquids (Figure 5C and Table 5).

These results show that the effect of using PG and dextran in combination (that is, the suppressing effect on cell death caused by freezing and thawing of mammalian cells) is exerted regardless of the type of the base liquid of the liquid for cryopreserving a cell.

**[Table 5]**

| Liquid for cryopreserving a cell | Immediately after freezing and thawing |
|---|---|
| | Cell viability (%) |
| PG/S | 81.7±11.4 |
| PG/R | 79.9±7.2 |
| PG/AR | 66.6±12.3 |
| PG/LR | 77.5±16.7 |
| PG+D/S | 91.9±5.2 |
| PG+D/R | 96.0±1.0 |
| PG+D/AR | 92.7±0.9 |
| PG+D/LR | 94.1±1.7 |

| | Viable cell recovery rate (%) |
|---|---|
| PG/S | 88.6±13.8 |
| PG/R | 72.1±11.1 |
| PG/AR | 71.6±9.3 |
| PG/LR | 86.1±15.2 |
| PG+D/S | 96.2±6.3 |
| PG+D/R | 85.4±9.7 |
| PG+D/AR | 100.9±7.1 |
| PG+D/LR | 104.0±8.3 |

| | Annexin V positivity rate (%) |
|---|---|
| PG/S | 24.2±7.6 |
| PG/R | 30.9±9.7 |
| PG/AR | 29.6±13.2 |
| PG/LR | 25.3±7.4 |
| PG+D/S | 12.6±0.9 |
| PG+D/R | 16.5±4.8 |
| PG+D/AR | 15.6±3.5 |
| PG+D/LR | 14.7±6.2 |

Figure 5 was created based on the results in Table 5.

In addition, as a result of culturing the hAD-MSCs after freezing and thawing for 1 to 7 days and analyzing changes in the number of cells over time, it was shown that the hAD-MSCs cryopreserved in the above 4 base liquids comprising 5% PG and 4.75% dextran proliferated more efficiently than the hAD-MSCs cryopreserved in the above 4 base liquids comprising 5% PG (Figure 6 and Table 6).

This result shows that the effect of using PG and dextran in combination (that is, the effect that increases the proliferation ability of mammalian cells after freezing and thawing) is exerted regardless of the type of the base liquid of the liquid for cryopreserving a cell.

**[Table 6]**

| Liquid for cryopreserving a cell | Number of cells (×10⁴ cells/well) | | | | |
|---|---|---|---|---|---|
| | Immediately after freezing and thawing | After freezing and thawing | | | |
| | | Day 1 | Day 3 | Day 5 | Day 7 |
| PG/S | 4.7±0.3 | 4.3±1.4 | 18.3±4.4 | 32.1±1.3 | 33.6±6.0 |
| PG/R | 5.1±0.5 | 4.1±1.7 | 13.6±3.3 | 27.9±2.5 | 30.9±3.9 |
| PG/AR | 4.9±0.5 | 3.4±1.2 | 12.9±5.5 | 28.8±3.4 | 30.9±1.6 |
| PG/LR | 5.3±0.4 | 3.9±0.6 | 14.2±3.1 | 30.1±7.6 | 30.9±3.3 |
| PG+D/S | 5.0±0.4 | 4.7±1.2 | 21.2±0.6 | 36.3±2.4 | 39.6±1.8 |
| PG+D/R | 4.9±0.7 | 4.1±0.9 | 17.6±1.9 | 35.8±4.8 | 34.8±1.2 |
| PG+D/AR | 5.2±0.3 | 5.4±1.2 | 18.3±5.0 | 36.8±9.7 | 35.5±3.4 |
| PG+D/LR | 4.9±0.6 | 4.2±0.8 | 18.3±2.6 | 34.0±2.2 | 38.6±3.8 |

Figure 6 was created based on the results in Table 6.

### 2-5 Study 5

In order to analyze the optimal concentration of PG in a liquid for cryopreserving a cell comprising PG, hAD-MSCs were cryopreserved in 8 liquids for cryopreserving a cell (LR containing 1.25% PG and 4.94% dextran; LR containing 2.5% PG and 4.88% dextran; LR containing 3.75% PG and 4.81% dextran; LR containing 5% PG and 4.75% dextran; LR containing 6.25% PG and 4.69% dextran; LR containing 7.5% PG and 4.63% dextran; LR containing 8.75% PG and 4.56% dextran; and LR containing 10% PG and 4.5% dextran), and the cell viability, the viable cell recovery rate, and the annexin V positivity rate of the hAD-MSCs immediately after freezing and thawing, and the cell proliferation ability thereof after freezing and thawing were analyzed.

As a result, when hAD-MSCs were cryopreserved in 6 liquids for cryopreserving a cell comprising 2.5 to 8.75% PG (LR containing 2.5% PG and 4.88% dextran; LR containing 3.75% PG and 4.81% dextran; LR containing 5% PG and 4.75% dextran; LR containing 6.25% PG and 4.69% dextran; LR containing 7.5% PG and 4.63% dextran; and LR containing 8.75% PG and 4.56% dextran), the cell viability and the viable cell recovery rate of the hAD-MSCs immediately after freezing and thawing were higher than when hAD-MSCs were cryopreserved in the liquid for cryopreserving a cell comprising 1.25% PG (LR containing 1.25% PG and 4.94% dextran) or the liquid for cryopreserving a cell comprising 10% PG (LR containing 10% PG and 4.5% dextran), and in particular, when the concentration of PG in the liquid for cryopreserving a cell was 2.5 to 6.25%, the cell viability was significantly higher (Figures 7A and 7B and Table 7).

In addition, when hAD-MSCs are cryopreserved in the above 6 liquids for cryopreserving a cell comprising 2.5 to 8.75% PG, the annexin V positivity rate of the hAD-MSCs immediately after freezing and thawing was lower than when hAD-MSCs were cryopreserved in the liquid for cryopreserving a cell comprising 1.25% PG or the liquid for cryopreserving a cell comprising 10% PG, and in particular, when the concentration of PG in the liquid for cryopreserving a cell was 2.5 to 6.25%, the annexin V positivity rate was significantly lower (Figure 7C and Table 7).

These results show that when mammalian cells are cryopreserved in a liquid for cryopreserving a cell containing 2.5 to 8.75% (particularly 2.5 to 6.25%) PG and dextran, cell death caused by freezing and thawing of the mammalian cells can be more effectively suppressed than when mammalian cells are cryopreserved in a liquid for cryopreserving a cell containing PG having a concentration other than 2.5 to 8.75% (particularly 2.5 to 6.25%) and dextran.

**[Table 7]**

| Concentration of PG in PG + D | Immediately after freezing and thawin g |
|---|---|
| | Cell viability (%) |
| 1.25% | 77.6±6.4 |
| 2.50% | 93.1±2.8 |
| 3.75% | 93.9±3.2 |
| 5.00% | 94.1±2.3 |
| 6.25% | 92.2±3.1 |
| 7.50% | 91.8±2.5 |
| 8.75% | 89.8±3.2 |
| 10.0% | 85.1±0.7 |

| | Viable cell recovery rate (%) |
|---|---|
| 1.25% | 81.8±10.7 |
| 2.50% | 101.4±3.1 |
| 3.75% | 100.4±3.5 |
| 5.00% | 96.2±6.7 |
| 6.25% | 92.5±7.7 |
| 7.50% | 93.5±4.7 |
| 8.75% | 89.8±10.5 |
| 10.0% | 91.6±7.8 |

| | Annexin V positivity rate (%) |
|---|---|
| 1.25% | 70.4±5.9 |
| 2.50% | 17.0±3.9 |
| 3.75% | 13.3±2.6 |
| 5.00% | 14.0±2.2 |
| 6.25% | 14.2±3.0 |
| 7.50% | 15.8±2.3 |
| 8.75% | 18.5±3.8 |
| 10.0% | 22.6±3.5 |

Figure 7 was created based on the results in Table 7.

In addition, as a result of culturing the hAD-MSCs after freezing and thawing for 1 to 7 days and analyzing changes in the number of cells over time, it was shown that from day 5 of culture onward, the hAD-MSCs cryopreserved in 5 liquids for cryopreserving a cell comprising 2.5 to 7.5% PG (LR containing 2.5% PG and 4.88% dextran; LR containing 3.75% PG and 4.81% dextran; LR containing 5% PG and 4.75% dextran; LR containing 6.25% PG and 4.69% dextran; and LR containing 7.5% PG and 4.63% dextran) proliferated more efficiently than the hAD-MSCs cryopreserved in the liquid for cryopreserving a cell comprising 10% PG (LR containing 10% PG and 4.5% dextran), and it was shown that in particular, when the concentration of PG in the liquid for cryopreserving a cell was 2.5 to 5%, the hAD-MSCs significantly proliferated (Figure 8 and Table 8).

This result shows that when mammalian cells are cryopreserved in a liquid for cryopreserving a cell containing 2.5 to 5% PG and dextran, the proliferation ability of the mammalian cells after freezing and thawing can be more effectively increased than when the mammalian cells are cryopreserved in a liquid for cryopreserving a cell containing PG having a concentration of other than 2.5 to 5% and dextran.

**[Table 8]**

| Concentration of PG in PG + D (%) | Number of cells (×10⁴ cells/well) | | | | |
|---|---|---|---|---|---|
| | Immediately after freezing and thawing | After freezing and thawing | | | |
| | | Day 1 | Day 3 | Day 5 | Day 7 |
| 1.25% | 4.9±0.5 | 1.4±0.4 | 10.5±3.9 | 30.3±10.7 | 40.1±8.4 |
| 2.50% | 5.1±0.6 | 4.2±1.2 | 18.3±3.0 | 44.8±5.5 | 53.8±4.7 |
| 3.75% | 4.8±0.3 | 4.3±1.2 | 19.3±1.6 | 51.0±6.5 | 56.1±8.4 |
| 5.00% | 4.8±0.4 | 3.9±0.8 | 20.0±2.2 | 50.4±1.4 | 51.6±1.3 |
| 6.25% | 4.8±0.2 | 4.7±0.7 | 18.6±3.3 | 41.3±2.8 | 43.3±4.1 |
| 7.50% | 4.8±0.2 | 4.2±0.8 | 19.5±2.4 | 39.5±2.4 | 41.8±3.8 |
| 8.75% | 4.7±0.5 | 4.4±0.8 | 17.6±0.6 | 36.1 ±1.7 | 37.8±1.4 |
| 10.0% | 4.9±0.1 | 3.8±0.6 | 15.1 ±1.4 | 34.8±1.5 | 37.9±5.2 |

Figure 8 was created based on the results in Table 8.

2-6 Study 6

In order to study the optimal concentration of dextran in a liquid for cryopreserving a cell comprising 2.5 to 5% PG and dextran, hAD-MSCs were cryopreserved in 6 liquids for cryopreserving a cell (LR containing 4% PG; LR containing 4% PG and 1.2% dextran; LR containing 4% PG and 2.4% dextran; LR containing 4% PG and 4.8% dextran; LR containing 4% PG and 7.2% dextran; and LR containing 4% PG and 9.6% dextran), and the cell viability, the viable cell recovery rate, and the annexin V positivity rate of the hAD-MSCs immediately after thawing were analyzed.

As a result, when hAD-MSCs were cryopreserved in the liquids for cryopreserving a cell containing 4% PG and 1.2 to 9.6% dextran (LR containing 4% PG and 1.2% dextran; LR containing 4% PG and 2.4% dextran; LR containing 4% PG and 4.8% dextran; LR containing 4% PG and 7.2% dextran; and LR containing 4% PG and 9.6% dextran), the cell viability and the viable cell recovery rate of the hAD-MSCs immediately after freezing and thawing were higher than when hAD-MSCs were cryopreserved in a liquid for cryopreserving a cell containing 4% PG without dextran (LR containing 4% PG), and in particular, when the concentration of dextran in the liquid for cryopreserving a cell was 1.2 to 9.6%, the cell viability was significantly higher, and when the concentration of dextran in the liquid for cryopreserving a cell was 2.4 to 9.6%, the viable cell recovery rate was significantly higher (Figures 9A and 9B and Table 9).

In addition, when hAD-MSCs were cryopreserved in the liquid for cryopreserving a cell containing 4% PG and 1.2 to 9.6% dextran, the annexin V positivity rate of the hAD-MSCs immediately after freezing and thawing was lower than when hAD-MSCs were cryopreserved in the liquid for cryopreserving a cell containing 4% PG without dextran, and in particular, when the concentration of dextran in the liquid for cryopreserving a cell was 4.8 to 9.6%, the annexin V positivity rate was significantly lower (Figures 9A and 9B Table 9).

These results show that when mammalian cells are cryopreserved in a liquid for cryopreserving a cell comprising 2.5 to 5% PG and at least 1.2% (particularly at least 2.4% [for example, 2.4 to 9.6% or 4.8 to 9.6%]) dextran, cell death caused by freezing and thawing of the mammalian cells can be more effectively suppressed than when mammalian cells are cryopreserved in a liquid for cryopreserving a cell containing 2.5 to 5% PG without dextran.

**[Table 9]**

| Liquid for cryopreserving a cell (Concentration of D in 4% PG + D) | Immediately after freezing and thawing |
|---|---|
| | Cell viability (%) |
| 0.0% | 73.7±9.2 |
| 1.2% | 85.7±5.1 |
| 2.4% | 91.5±3.5 |
| 4.8% | 95.6±1.5 |
| 7.2% | 95.8±0.8 |
| 9.6% | 96.2±0.4 |

| | Viable cell recovery rate (%) |
|---|---|
| 0.0% | 61.3±10.9 |
| 1.2% | 76.4±6.6 |
| 2.4% | 90.6±9.9 |
| 4.8% | 99.4±5.3 |
| 7.2% | 98.7±9.2 |
| 9.6% | 96.4±8.0 |

| | Annexin V positivity rate (%) |
|---|---|
| 0.0% | 27.8±3.7 |
| 1.2% | 26.5±3.2 |
| 2.4% | 23.7±3.8 |
| 4.8% | 19.1±2.9 |
| 7.2% | 15.3±3.8 |
| 9.6% | 14.9±3.5 |

Figure 9 was created based on the results in Table 9.

### 2-7 Study 7

In order to study whether the same effect as when dextran and PG were used in combination was also exerted when HES, as a polymer compound other than dextran, and PG were used in combination, hAD-MSCs were cryopreserved in 6 liquids for cryopreserving a cell (LR containing 4% PG; LR containing 10% PG; LR containing 4% PG and 4.8% dextran; LR containing 10% PG and 4.5% dextran; LR containing 4% PG and 4.8% HES; and LR containing 10% PG and 4.5% HES), and the cell viability, the viable cell recovery rate, and the annexin V positivity rate of the hAD-MSCs immediately after freezing and thawing were analyzed.

As a result, when hAD-MSCs were cryopreserved in the liquids for cryopreserving a cell comprising PG and HES (LR containing 4% PG and 4.8% HES; and LR containing 10% PG and 4.5% HES), the cell viability and the viable cell recovery rate of the hAD-MSCs immediately after freezing and thawing were higher than when hAD-MSCs were cryopreserved in the liquids for cryopreserving a cell comprising only PG (LR containing 4% PG; and LR containing 10% PG), and the annexin V positivity rate was lower, as was the case when hAD-MSCs were cryopreserved in the liquids for cryopreserving a cell comprising PG and dextran (LR containing 4% PG and 4.8% dextran; and LR containing 10% PG and 4.5% dextran) (Figure 10 and Table 10). Similarly, when the concentration of PG in the liquid for cryopreserving a cell comprising PG and HES was 4%, the cell viability and the viable cell recovery rate of the hAD-MSCs immediately after freezing and thawing were higher, and the annexin V positivity rate was lower than when the concentration of PG was 10% (Figure 10 and Table 10).

These results show that when mammalian cells are cryopreserved in a liquid for cryopreserving a cell comprising PG and HES (a polymer compound similar to dextran), cell death caused by freezing and thawing of the mammalian cells can be effectively suppressed, as was the case when mammalian cells are cryopreserved in a liquid for cryopreserving a cell containing PG and dextran.

**[Table 10]**

| Liquid for cryopreserving a cell | Immediately after freezing and thawing |
|---|---|
| | Cell viability (%) |
| 4%PG | 78.4±16.9 |
| 10%PG | 70.1±3.8 |
| 4%PG+D | 92.3±2.8 |
| 10%PG+D | 86.0±7.8 |
| 4%PG+HES | 95.3±1.3 |
| 10%PG+HES | 84.3±5.5 |

| | Viable cell recovery rate (%) |
|---|---|
| 4%PG | 74.5±14.9 |
| 10%PG | 67.2±2.0 |
| 4%PG+D | 87.4±8.3 |
| 10%PG+D | 82.6±7.0 |
| 4%PG+HES | 89.3±1.6 |
| 10%PG+HES | 73.4±7.0 |

| | Annexin V positivity rate (%) |
|---|---|
| 4%PG | 35.7±8.6 |
| 10%PG | 43.4±2.9 |
| 4%PG+D | 18.4±0.9 |
| 10%PG+D | 41.3±8.2 |
| 4%PG+HES | 18.2±2.6 |
| 10%PG+HES | 39.8±7.6 |

Figure 10 was created based on the results in Table 10.

In addition, as a result of culturing the hAD-MSCs after freezing and thawing for 1 to 7 days and analyzing changes in the number of cells over time, when carrying out analysis using the above 6 liquids for cryopreserving a cell comprising trehalose, that is, LR containing 4% PG and 2.88% trehalose; LR containing 10% PG and 2.7% trehalose; LR containing 4% PG, 2.88% trehalose, and 4.8% dextran; LR containing 10% PG, 2.7% trehalose, and 4.5% dextran; LR containing 4% PG, 2.88% trehalose, and 4.8% HES; and LR containing 10% PG, 2.7% trehalose, and 4.5% HES, the effect of use of PG in combination with HES was similarly observed (Figure 11 and Table 11).

These results show that the effect of using PG and a polymer compound (dextran, HES, or the like) in combination (that is, the effect that increases the proliferation ability of mammalian cells after freezing and thawing) is also exerted when PG is used in combination with trehalose.

**[Table 11]**

| Liquid for cryopreserving a cell | Immediately after freezing and thawing |
|---|---|
| | Cell viability (%) |
| 4%PG+Tre | 81.6±6.9 |
| 10%PG+Tre | 64.9±9.0 |
| 4%PG+Tre+D | 91.9±3.2 |
| 10%PG+Tre+D | 85.9±4.2 |
| 4%PG+Tre+HES | 90.8±7.0 |
| 10%PG+Tre+HES | 87.1±3.0 |

| | Viable cell recovery rate (%) |
|---|---|
| 4%PG+Tre | 77.6±4.8 |
| 10%PG+Tre | 58.3±9.2 |
| 4%PG+Tre+D | 90.1 ±8.1 |
| 10%PG+Tre+D | 81.6±1.2 |
| 4%PG+Tre+HES | 96.0±11.0 |
| 10%PG+Tre+HES | 83.3±2.6 |

| | Annexin V positivity rate (%) |
|---|---|
| 4%PG+Tre | 28.1±2.8 |
| 10%PG+Tre | 39.9±7.6 |
| 4%PG+Tre+D | 16.3±3.7 |
| 10%PG+Tre+D | 28.0±4.9 |
| 4%PG+Tre+HES | 13.1±2.9 |
| 10%PG+Tre+HES | 23.8±3.8 |

Figure 11 was created based on the results in Table 11.

2-8 Study 8

In order to confirm the effect of using PG in combination with trehalose or dextran in a liquid for cryopreserving a cell, hAD-MSCs were cryopreserved in 3 liquids for cryopreserving a cell (LR containing 2.5% PG; LR containing 2.5% PG and 2.925% trehalose; and LR containing 2.5% PG, 2.925% trehalose, and 4.875% dextran), and the cell viability, the viable cell recovery rate, and the annexin V positivity rate of the hAD-MSCs immediately after thawing were analyzed.

As a result, when hAD-MSCs were cryopreserved in LR containing 2.5% PG and 2.925% trehalose; or LR containing 2.5% PG, 2.925% trehalose, and 4.875% dextran, the cell viability and the viable cell recovery rate of the hAD-MSCs immediately after thawing were higher than when hAD-MSCs were cryopreserved in LR containing 2.5% PG, and in particular, when LR containing 2.5% PG, 2.925% trehalose, and 4.875% dextran was used, these values were significantly higher (Figures 12A and 12B and Table 12).

In addition, when hAD-MSCs were cryopreserved in LR containing 2.5% PG and 2.925% trehalose; or LR containing 2.5% PG, 2.925% trehalose, and 4.875% dextran, the annexin V positivity rate of the hAD-MSCs immediately after thawing was significantly lower than when hAD-MSCs were cryopreserved in LR containing 2.5% PG (Figure 12C and Table 12) .

These results show that cryopreservation of mammalian cells in a liquid for cryopreserving a cell consisting of lactated Ringer's solution comprising PG, trehalose, and dextran has a more effective suppressing action on cell death caused by freezing and thawing of the mammalian cells than cryopreservation of mammalian cells in a liquid for cryopreserving a cell consisting of lactated Ringer's solution comprising only PG.

**[Table 12]**

| Liquid for cryopreserving a cell | Before cryopreservation | Immediately after freezing and thawing |
|---|---|---|
| | Cell vi ability (%) | |
| PG | | 33.2±11.9 |
| PG+Tre | 97.6±1.4 | 44.0±15.0 |
| PG+Tre+D | | 80.8±5.9 |

| | Viable cell re covery rate (%) | |
|---|---|---|
| PG | | 32.2±11.4 |
| PG+Tre | 100.0 | 42.0±14.4 |
| PG+Tre+D | | 82.5±6.8 |

| | Annexin V positivity rate (%) | |
|---|---|---|
| PG | | 63.3±5.9 |
| PG+Tre | | 52.7±5.4 |
| PG+Tre+D | | 28.2±5.0 |

**Figure** 12 was created based on the results in Table 12.

### 2-9 Study 9

From the results of Study 8, the effect of use of PG in combination with trehalose was observed, and thus in order to study whether the effect of use of PG in combination with a sugar other than trehalose was observed, hAD-MSCs were cryopreserved in LR containing 4% PG comprising one of 5 sugars (1.44% glucose, 1.44% fructose, 2.88% trehalose, 2.88% sucrose, or 2.88% lactose), and the cell viability and the viable cell recovery rate of the hAD-MSCs immediately after thawing were analyzed. As a comparative control, LR containing 4% PG without these sugars was used to carry out the same analysis. In addition, the molar concentrations of all the sugars are the same, 0.08 M.

As a result, even when hAD-MSCs were cryopreserved in liquids for cryopreserving a cell containing 4% PG and comprising 4 sugars, respectively, other than trehalose (glucose, fructose, sucrose, or lactose), the cell viability and the viable cell recovery rate of the hAD-MSCs immediately after freezing and thawing were almost the same as when hAD-MSCs were cryopreserved in the liquid for cryopreserving a cell containing 4% PG (Figure 13 and Table 13) .

This result shows that the effect of use of PG in combination with sugars other than trehalose is not observed.

**[Table 13]**

| Liquid for cryopreserving a cell | Immediately after freezing and thawing |
|---|---|
| | Cell viability (%) |
| 4%PG | 79.8±5.6 |
| 4%PG+Glu | 83.3±6.5 |
| 4%PG+Flu | 84.2±2.1 |
| 4%PG+Tre | 89.4±4.6 |
| 4%PG+Suc | 80.1±8.0 |
| 4%PG+Lac | 82.5±4.5 |

| | Viable cell recovery rate (%) |
|---|---|
| 4%PG | 76.8±5.0 |
| 4%PG+Glu | 79.0±12.3 |
| 4%PG+Flu | 90.7±3.3 |
| 4%PG+Tre | 99.0±6.2 |
| 4%PG+Suc | 83.0±3.5 |
| 4%PG+Lac | 88.6±10.5 |

**Figure** 13 was created based on the results in Table 13.

### 2-10 Study 10

In order to study the optimal concentration of trehalose in a liquid for cryopreserving a cell comprising 2.5 to 5% PG and trehalose, hAD-MSCs were cryopreserved in 6 liquids for cryopreserving a cell (LR containing 4% PG; LR containing 4% PG and 0.72% trehalose; LR containing 4% PG and 1.44% trehalose; LR containing 4% PG and 2.88% trehalose; LR containing 4% PG and 5.76% trehalose; and LR containing 4% PG and 11.52% trehalose), and the cell viability, the viable cell recovery rate, and the annexin V positivity rate of the hAD-MSCs immediately after thawing were analyzed.

As a result, when hAD-MSCs were cryopreserved in liquids for cryopreserving a cell containing 4% PG and 0.72 to 11.52% trehalose (LR containing 4% PG and 0.72% trehalose; LR containing 4% PG and 1.44% trehalose; LR containing 4% PG and 2.88% trehalose; LR containing 4% PG and 5.76% trehalose; and LR containing 4% PG and 11.52% trehalose), the cell viability and the viable cell recovery rate of the hAD-MSCs immediately after freezing and thawing were higher than when hAD-MSCs were cryopreserved in a liquid for cryopreserving a cell containing 4% PG without trehalose (LR containing 4% PG), and in particular, when the concentration of trehalose in the liquid for cryopreserving a cell was 1.44 to 2.88% and 11.52%, the cell viability was significantly higher, and when the concentration of trehalose in the liquid for cryopreserving a cell was 0.72 to 5.76%, the viable cell recovery rate was significantly higher (Figures 14A and 14B and Table 14).

In addition, when hAD-MSCs were cryopreserved in the liquids for cryopreserving a cell containing 4% PG and 0.72 to 11.52% trehalose, the annexin V positivity rate of the hAD-MSCs immediately after freezing and thawing was lower than when hAD-MSCs were cryopreserved in the liquid for cryopreserving a cell containing 4% PG without trehalose (Figure 14C and Table 14).

These results show that when mammalian cells are cryopreserved in a liquid for cryopreserving a cell comprising 2.5 to 5% PG and at least 0.72% (for example, 1.44 to 11.52%, or 0.72 to 5.76%) trehalose, cell death caused by freezing and thawing of the mammalian cells can be more effectively suppressed than when mammalian cells are cryopreserved in a liquid for cryopreserving a cell containing 2.5 to 5% PG without trehalose.

**[Table 14]**

| Liquid for cryopreserving a cell (Concentration of Tre in 4% PG + Tre) | Immediately after freezing and thawing |
|---|---|
| | Cell viability (%) |
| 0.00% | 72.9±10.1 |
| 0.72% | 82.1±3.7 |
| 1.44% | 85.5±1.7 |
| 2.88% | 87.8±3.6 |
| 5.76% | 82.5±5.2 |
| 11.52% | 86.4±5.1 |

| | Viable cell recovery rate (%) |
|---|---|
| 0.00% | 61.6±6.7 |
| 0.72% | 81.7±11.2 |
| 1.44% | 91.2±9.4 |
| 2.88% | 95.0±8.0 |
| 5.76% | 83.2±8.9 |
| 11.52% | 76.9±11.4 |

| | Annexin V positivity rate (%) |
|---|---|
| 0.00% | 29.1±6.0 |
| 0.72% | 25.2±3.4 |
| 1.44% | 24.0±2.3 |
| 2.88% | 28.0±3.6 |
| 5.76% | 23.7±6.7 |
| 11.52% | 23.0±5.4 |

**Figure** 14 was created based on the results in Table 14.

### 2-11 Study 11

In order to confirm that the effect of use of PG in combination with trehalose was also exerted when mammalian cells other than hAD-MSCs were cryopreserved in the present cryopreservation liquid, hCD8-positive T cells were cryopreserved in 3 liquids for cryopreserving a cell (culture medium containing serum and comprising 4% PG; LR containing trehalose and comprising 4% PG; and LR comprising 4% PG), and the cell viability and the viable cell recovery rate of the hCD8-positive T cells immediately after thawing, and 1 hour, 3 hours, and 6 hours after thawing were analyzed.

As a result, when hCD8-positive T cells were cryopreserved in the culture medium containing serum and comprising 4% PG or LR containing trehalose and comprising 4% PG, the cell viability and the viable cell recovery rate of the hCD8-positive T cells immediately after thawing or thereafter were higher than when hCD8-positive T cells were cryopreserved in LR comprising 4% PG (Figure 15 and Table 15) .

This result shows that the lactated Ringer's solution containing trehalose and comprising PG has a cell preservation effect comparable to that of the culture medium containing serum and comprising PG, and also shows that the lactated Ringer's solution containing trehalose and comprising PG has a higher cell preservation effect than that of the lactated Ringer's solution comprising PG, supporting the results of Studies 7 and 8 described above.

**[Table 15]**

| Liquid for cryopreserving a cell | Before cryopreservation | After cryopreservation and thawing | | | |
|---|---|---|---|---|---|
| | | 0 Hours | 1 Hour | 3 Hours | 6 Hours |
| | Cell viability (%) | | | | |
| PG+Med | 87.0±1.7 | 52.5±5.6 | 49.1±8.8 | 50.7±4.2 | 47.3±5.2 |
| PG+Tre | 79.7±1.1 | 59.5±2.4 | 43.6±6.7 | 55.8±4.7 | 51.4±3.9 |
| PG | 78.6±1.0 | 48.0±11.7 | 22.5±6.1 | 30.6±4.4 | 35.7±8.3 |

| | Viable cell recovery rate (%) | | | | |
|---|---|---|---|---|---|
| PG+Med | - | 60.1±6.4 | 54.9±8.2 | 56.9±8.5 | 49.3±6.6 |
| PG+Tre | - | 67.2±8.3 | 48.6±14.5 | 72.9±12.2 | 68.9±17.2 |
| PG | - | 50.7±18.0 | 23.6±8.9 | 36.3±5.4 | 44.5±12.3 |

Figure 15 was created based on the results in Table 15.

### 2-12 Study 12

In order to confirm that the effects shown in Studies 1 to 10 described above were also exerted when mammalian cells other than hAD-MSCs were cryopreserved in the present cryopreservation liquid, hCD8-positive T cells were cryopreserved in 5 liquids for cryopreserving a cell (LR containing 2% PG, 2.7% trehalose, and 4.5% dextran; LR containing 3% PG, 2.7% trehalose, and 4.5% dextran; LR containing 4% PG, 2.7% trehalose, and 4.5% dextran; LR containing 5% PG, 2.7% trehalose, and 4.5% dextran; and LR containing 7.5% PG, 2.7% trehalose, and 4.5% dextran), and the cell viability and the viable cell recovery rate of the hCD8-positive T cells immediately after thawing and 3 hours after thawing were analyzed.

As a result, when hCD8-positive T cells were cryopreserved in 4 liquids for cryopreserving a cell comprising 3 to 7.5% PG (LR containing 3% PG, 2.7% trehalose, and 4.5% dextran; LR containing 4% PG, 2.7% trehalose, and 4.5% dextran; LR containing 5% PG, 2.7% trehalose, and 4.5% dextran; and LR containing 7.5% PG, 2.7% trehalose, and 4.5% dextran), the cell viability and the viable cell recovery rate of the hCD8-positive T cells immediately after thawing or thereafter were higher than when hCD8-positive T cells were cryopreserved in a liquid for cryopreserving a cell comprising 2% PG (LR containing 2% PG, 2.7% trehalose, and 4.5% dextran), and in particular, when the concentration of PG in the liquid for cryopreserving a cell was 3 to 5%, the cell viability was higher, and when the concentration of PG in the liquid for cryopreserving a cell was 4 to 5%, the viable cell recovery rate was higher (Figure 16 and Table 16) .

This result shows that when mammalian cells are cryopreserved in a liquid for cryopreserving a cell containing 3 to 7.5% (particularly 3 to 5%, and even 4 to 5%) PG, trehalose, and dextran, cell death caused by freezing and thawing of the mammalian cells can be more effectively suppressed than when mammalian cells are cryopreserved in a liquid for cryopreserving a cell containing PG having a concentration other than 3 to 7.5% (particularly 3 to 5%, and even 4 to 5%), trehalose, and dextran, supporting the results of Studies 1 to 10 described above.

**[Table 16]**

| Liquid for cryopreserving a cell (Concentration of PG in PG + Tre + D) | Before cryo preservation | After cryopreservation and thawing | |
|---|---|---|---|
| | | 0 Hours | 3 Hours |
| | Cell viability (%) | | |
| 2% | 80.3±1.2 | 62.3±10.4 | 63.1±2.0 |
| 3% | 80.0±1.4 | 73.5±2.6 | 73.7±0.9 |
| 4% | 78.4±2.0 | 75.6±3.4 | 74.8±1.5 |
| 5% | 79.2±0.8 | 75.9±3.4 | 75.0±1.2 |
| 7.5% | 79.2±1.8 | 68.6±4.3 | 72.9±3.6 |

| | Via ble cell recovery rate (%) | | |
|---|---|---|---|
| 2% | - | 68.8±19.9 | 73.8±5.5 |
| 3% | - | 77.8±11.7 | 87.7±6.6 |
| 4% | - | 88.1±22.1 | 91.0±10.6 |
| 5% | - | 83.2±23.6 | 90.4±8.0 |
| 7.5% | - | 57.9±8.9 | 80.7±9.3 |

Figure 16 was created based on the results in Table 16.

### 2-13 Study 13

Following Study 12, in order to confirm that the effects shown in Studies 1 to 10 described above were also exerted when mammalian cells other than hAD-MSCs were cryopreserved in the present cryopreservation liquid, hCD8-positive T cells were cryopreserved in the 5 liquids for cryopreserving a cell used in Study 12, and the cell viability, the viable cell recovery rate, and the annexin V positivity rate of the hCD8-positive T cells immediately after thawing were analyzed.

As a result, when hCD8-positive T cells were cryopreserved in 4 liquids for cryopreserving a cell comprising 3 to 7.5% PG (LR containing 3% PG, 2.7% trehalose, and 4.5% dextran; LR containing 4% PG, 2.7% trehalose, and 4.5% dextran; LR containing 5% PG, 2.7% trehalose, and 4.5% dextran; and LR containing 7.5% PG, 2.7% trehalose, and 4.5% dextran), the cell viability and the viable cell recovery rate of the hCD8-positive T cells immediately after freezing and thawing were higher than when hCD8-positive T cells were cryopreserved in the liquid for cryopreserving a cell comprising 2% PG of PG (LR containing 2% PG, 2.7% trehalose, and 4.5% dextran), and in particular, when the concentration of PG in the liquid for cryopreserving a cell was 4 to 5%, the cell viability was higher, and when the concentration of PG in the liquid for cryopreserving a cell was 3 to 5%, the viable cell recovery rate was higher (Figures 17A and 17B and Table 17).

In addition, when hCD8-positive T cells were cryopreserved in the above 4 liquids for cryopreserving a cell comprising 3 to 7.5% PG, the annexin V positivity rate of the hCD8-positive T cells immediately after freezing and thawing was significantly lower than when hCD8-positive T cells were cryopreserved in the above liquid for cryopreserving a cell comprising 2% PG of PG, and in particular, when the concentration of PG in the liquid for cryopreserving a cell was 4 to 5%, the annexin V positivity rate was lowest (Figure 17C and Table 17).

These results show that when mammalian cells are cryopreserved in a liquid for cryopreserving a cell containing 3 to 7.5% (particularly 3 to 5%, and even 4 to 5%) PG, trehalose, and dextran, cell death caused by freezing and thawing of the mammalian cells can be more effectively suppressed than when mammalian cells are cryopreserved in a liquid for cryopreserving a cell containing PG having a concentration other than 3 to 7.5% (particularly 3 to 5%, and even 4 to 5%), trehalose, and dextran, supporting the results of Studies 1 to 10 described above.

**[Table 17]**

| Liquid for cryopreserving a cell (Concentration of PG in PG + Tre + D) | Immediately after freezing and thawing |
|---|---|
| | Cell viability (%) |
| 2% | 77.4±5.0 |
| 3% | 83.0±7.9 |
| 4% | 86.1±3.0 |
| 5% | 86.9±4.3 |
| 7.5% | 82.5±3.8 |

| | Viable cell recovery rate (%) |
|---|---|
| 2% | 86.8±8.5 |
| 3% | 99.3±14.5 |
| 4% | 102.4±6.4 |
| 5% | 96.6±14.4 |
| 7.5% | 88.8±8.2 |

| | Annexin V positivity rate (%) |
|---|---|
| 2% | 42.3±1.9 |
| 3% | 31.9±1.7 |
| 4% | 26.8±2.5 |
| 5% | 26.0±0.8 |
| 7.5% | 31.0±4.8 |

Figure 17 was created based on the results in Table 17.

### 2-14 Study 14

Following Studies 12 and 13, in order to confirm that the effects shown in Studies 1 to 10 described above were also exerted when mammalian cells other than hAD-MSCs were cryopreserved in the present cryopreservation liquid, hCD4-positive T cells were cryopreserved in the 5 liquids for cryopreserving a cell used in Studies 12 and 13, and the cell viability, the viable cell recovery rate, and the annexin V positivity rate of the hCD4-positive T cells immediately after thawing were analyzed.

As a result, when hCD4-positive T cells were cryopreserved in 4 liquids for cryopreserving a cell comprising 3 to 7.5% PG (LR containing 3% PG, 2.7% trehalose, and 4.5% dextran; LR containing 4% PG, 2.7% trehalose, and 4.5% dextran; LR containing 5% PG, 2.7% trehalose, and 4.5% dextran; and LR containing 7.5% PG, 2.7% trehalose, and 4.5% dextran), the cell viability and the viable cell recovery rate of the hCD4-positive T cells immediately after freezing and thawing were significantly higher than when hCD4-positive T cells were cryopreserved in the liquid for cryopreserving a cell comprising 2% PG of PG (LR containing 2% PG, 2.7% trehalose, and 4.5% dextran), and in particular, when the concentration of PG in the liquid for cryopreserving a cell was 4 to 5%, the cell viability and the viable cell recovery rate were highest (Figures 18A and 18B and Table 18).

In addition, when hCD4-positive T cells were cryopreserved in the above 4 liquids for cryopreserving a cell comprising 3 to 7.5% PG, the annexin V positivity rate of the hCD4-positive T cells immediately after freezing and thawing was significantly lower than when hCD4-positive T cells were cryopreserved in the above liquid for cryopreserving a cell comprising 2% PG of PG, and in particular, when the concentration of PG in the liquid for cryopreserving a cell was 4 to 5%, the annexin V positivity rate was lowest (Figure 18C and Table 18).

These results show that when mammalian cells are cryopreserved in a liquid for cryopreserving a cell containing 3 to 7.5% (particularly 3 to 5%, and even 4 to 5%) PG, trehalose, and dextran, cell death caused by freezing and thawing of the mammalian cells can be more effectively suppressed than when mammalian cells are cryopreserved in a liquid for cryopreserving a cell containing PG having a concentration other than 3 to 7.5% (particularly 3 to 5%, and even 4 to 5%), trehalose, and dextran, supporting the results of Studies 1 to 13 described above.

**[Table 18]**

| Liquid for cryopreserving a cell (Concentration of PG in PG + Tre + D) | Immediately after freezing and thawing |
|---|---|
| | Cell viability (%) |
| 2% | 58.8±12.4 |
| 3% | 80.4±4.8 |
| 4% | 85.3±5.6 |
| 5% | 89.0±4.8 |
| 7.5% | 78.7±9.4 |

| | Viable cell recovery rate (%) |
|---|---|
| 2% | 60.8±12.7 |
| 3% | 95.1±5.5 |
| 4% | 102.0±7.8 |
| 5% | 104.6±5.1 |
| 7.5% | 84.2±12.1 |

| | Annexin V positivity rate (%) |
|---|---|
| 2% | 40.1 ±8.7 |
| 3% | 24.2±4.9 |
| 4% | 21.0±4.1 |
| 5% | 20.0±3.1 |
| 7.5% | 24.8±4.5 |

Figure 18 was created based on the results in Table 18.

2-15 Study 15

Following studies 12 to 14, in order to confirm that the effects shown in Studies 1 to 10 described above were also exerted when mammalian cells other than hAD-MSCs were cryopreserved in the present cryopreservation liquid, hCD34-positive hematopoietic stem cells (the cell viability before cryopreservation was 73.3 ± 1.2%) were cryopreserved in the 5 liquids for cryopreserving a cell used in Studies 12 to 14, and the cell viability and the CD34 positivity rate of the hCD4-positive T cells immediately after thawing were analyzed.

As a result, when hCD34-positive hematopoietic stem cells were cryopreserved in 4 liquids for cryopreserving a cell comprising 3 to 7.5% PG (LR containing 3% PG, 2.7% trehalose, and 4.5% dextran; LR containing 4% PG, 2.7% trehalose, and 4.5% dextran; LR containing 5% PG, 2.7% trehalose, and 4.5% dextran; and LR containing 7.5% PG, 2.7% trehalose, and 4.5% dextran), the cell viability of the hCD34-positive hematopoietic stem cells immediately after thawing was higher than when hCD34-positive hematopoietic stem cells were cryopreserved in the liquid for cryopreserving a cell comprising 2% PG of PG (LR containing 2% PG, 2.7% trehalose, and 4.5% dextran), and in particular, when the concentration of PG in the liquid for cryopreserving a cell was 3 to 5%, the cell viability was higher (Figure 19A and Table 19).

This result shows that when mammalian cells are cryopreserved in a liquid for cryopreserving a cell containing 3 to 7.5% (particularly 3 to 5%) PG, trehalose, and dextran, cell death caused by freezing and thawing of the mammalian cells can be more effectively suppressed than when mammalian cells are cryopreserved in a liquid for cryopreserving a cell containing PG having a concentration other than 3 to 7.5% (particularly 3 to 5%), trehalose, and dextran, supporting the results of Studies 1 to 14 described above.

In addition, the CD34 positivity rate in hCD34-positive hematopoietic stem cells after cryopreservation was almost the same as the CD34 positivity rate in hCD34-positive hematopoietic stem cells before cryopreservation (Figure 19B and Table 19), and thus it was found that PG, trehalose, and dextran comprised in the liquid for cryopreserving a cell do not affect the properties of hCD34-positive hematopoietic stem cells.

**[Table 19]**

| Liquid for cryopreserving a cell (Concentration of PG in PG + Tre + D) | Cell viability before cryopreservation (%) | Cell viability immediately after freezing and thawing (%) |
|---|---|---|
| 2% | 73.3±1.2 | 45.0±8.5 |
| 3% | | 54.4±5.7 |
| 4% | | 51.0±4.6 |
| 5% | | 54.8±6.6 |
| 7.5% | | 51.1±8.1 |

| Liquid for cryopreserving a cell (Concentration of PG in PG + Tre + D) | Ratio of CD34 positivity rate and thawing to that be immediately after freezing fore cryopreservation | |
|---|---|---|
| 2% | 1.0± 0.4 | |
| 3% | 0.9± 0.4 | |
| 4% | 1.0± 0.3 | |
| 5% | 1.3± 0.2 | |
| 7.5% | 1.2± 0.7 | |

Figure 19 was created based on the results in Table 19.

### Industrial Applicability

According to the present invention, cell death caused by freezing and thawing of a mammalian cell can be effectively suppressed, and the proliferation ability of a mammalian cell after freezing and thawing can be effectively increased, and thus the present invention is useful in the field of transplantation medicine in regenerative medicine or the like and the field of cancer treatment.

## Claims

1. A liquid for cryopreserving a mammalian cell, comprising 2.5 to 8.75 (v/v)% propylene glycol.

2. The liquid according to claim 1, wherein a concentration of propylene glycol is 2.5 to 5.0 (v/v)%, and wherein the liquid is used to increase a proliferation ability of a mammalian cell after freezing and thawing.

3. The liquid according to claim 1, wherein the liquid further comprises a polymer compound selected from dextran or a derivative thereof or a salt of the dextran or the derivative; and hydroxyethyl starch or a derivative thereof or a salt of the hydroxyethyl starch or the derivative.

4. The liquid according to claim 1, wherein the liquid further comprises trehalose or a derivative thereof or a salt of the trehalose or the derivative.

5. The liquid according to claim 1, wherein the liquid is an isotonic solution comprising 2.5 to 8.75 (v/v)% propylene glycol.

6. The liquid according to claim 5, wherein the isotonic solution is selected from lactated Ringer's solution, physiological saline, Ringer's solution, and acetated Ringer's solution.

7. The liquid according to any one of claims 1 to 6, wherein the mammalian cell is a mesenchymal stem cell, a T cell, or a hematopoietic stem cell.

8. The liquid according to any one of claims 1 to 6, wherein the liquid comprises a mammalian cell.

9. The liquid according to claim 8, wherein the mammalian cell is a mesenchymal stem cell, a T cell, or a hematopoietic stem cell.

10. The liquid according to claim 9, wherein the liquid comprises umbilical cord blood.

11. A method for cryopreserving a mammalian cell, comprising step (a) of cryopreserving the liquid according to claim 8.

12. The method according to claim 11, wherein the method further comprises step (b) of freezing and thawing the mammalian cell and culturing the same for 5 days or more after step (a).
